(19) Europäisches Patentamt
European Patent Office
Office européen des brevets

(11) EP 4 169 512 A1

(12) DEMANDE DE BREVET EUROPEEN

(43) Date de publication:
**26.04.2023 Bulletin 2023/17**

(21) Numéro de dépôt: **22201948.1**

(22) Date de dépôt: **30.05.2013**

(51) Classification Internationale des Brevets (IPC):
***A61K 31/40*** (2006.01) ***A61K 31/402*** (2006.01)
***A61K 31/4015*** (2006.01) ***A61K 9/00*** (2006.01)
***A61K 9/107*** (2006.01) ***A61P 17/00*** (2006.01)

(52) Classification Coopérative des Brevets (CPC):
**A61K 31/4015; A61K 9/0014; A61K 9/107; A61K 31/40; A61K 31/402; A61P 17/00**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **01.06.2012 FR 1255090**
**01.06.2012 US 201261654687 P**

(62) Numéro(s) de document de la (des) demande(s) initiale(s) en application de l'article 76 CBE:
**13725982.6 / 2 872 116**

(27) Demande déposée antérieurement:
**13.05.2013 PCT/EP2013/061196**

(71) Demandeur: **Galderma Research & Development**
**06410 Biot (FR)**

(72) Inventeur: **DJEDOUR, Amel**
**06600 Antibes (FR)**

(74) Mandataire: **Nederlandsch Octrooibureau**
**P.O. Box 29720**
**2502 LS The Hague (NL)**

Remarques:
Cette demande a été déposée le 17-10-2022 comme demande- divisionnaire de la demande mentionnée sous le code INID 62.

# (54) COMPOSITION DERMATOLOGIQUE COMPRENANT DES OLÉOSOMES ET DES RÉTINOÏDES, SON PROCÉDÉ DE PRÉPARATION ET SON UTILISATION

(57) La présente invention se rapporte à des compositions comprenant au moins au moins l'acide 3"-tert-butyl-4'-(2-hydroxy-ethoxy)-4"-pyrrolidin-1-yl-[1,1';3',1 "]-terphenyl-4-carboxylique, caractérisée en ce que :
- ladite composition est une émulsion huile-dans-eau formée de globules liquides huileux pourvus chacun d'un enrobage cristal liquide lamellaire et dispersés dans une phase aqueuse,
- le rétinoïde est présent soit dans les globules liquides huileux, soit dans la phase aqueuse,
- chaque globule liquide huileux est unitairement enrobé d'une couche monolamellaire ou oligolamellaire obtenue à partir d'au moins un agent tensioactif lipophile, d'au moins un agent tensioactif hydrophile et d'au moins un tensioactif anionique distinct, et les globules liquides huileux enrobés ont un diamètre moyen inférieur à 800 nm.

EP 4 169 512 A1

## Description

La présente invention se rapporte à une composition pharmaceutique, notamment dermatologique, à base d'un rétinoïde, ledit rétinoïde étant présent sous forme solubilisée au sein d'oléosomes dispersés dans une phase aqueuse, ou directement solubilisé dans la phase aqueuse. Elle porte également sur son procédé de préparation et sur son utilisation pour traiter des affections dermatologiques, en particulier l'acné, les ichtyoses, les états ichtyosiformes, l'hyperkératose palmoplantaire ou le psoriasis, préférentiellement l'acné.

Les rétinoïdes sont des actifs couramment utilisés en dermatologie, mais ils sont connus comme étant pour la plupart des principes actifs irritants. Il est donc indispensable de trouver des compositions permettant de maintenir l'activité biologique, et donc l'efficacité, d'un rétinoïde, tout en diminuant au maximum son caractère irritant.

La recherche de nouvelles formulations permettant de maintenir l'activité pharmaceutique d'un rétinoïde, tout en améliorant sa tolérance, est donc d'un intérêt constant.

A cet effet, il a été proposé d'améliorer la tolérance des rétinoïdes par voie topique, en ajoutant des composés anti-irritants dans la composition.

La demande FR 2 894 820 décrit par exemple des formulations galéniques mettant en oeuvre des anti-irritants comme l'allantoïne, le disodium édétate, en association avec un rétinoïde particulier, l'adapalène.

D'autres technologies proposent de disperser le rétinoïde, à l'état libre ou en l'adsorbant sur des particules, afin d'améliorer sa tolérance. Par exemple, le brevet US 5,955,109 propose d'incorporer un rétinoïde dans des microsphères poreuses (Microsponge®), afin de diminuer la libération du rétinoïde dans les couches de la peau, ce qui engendre une diminution du niveau d'irritation par un contrôle de la cinétique de libération de l'actif au travers de la peau. De fait, un tel système comprenant le rétinoïde à l'état dispersé implique la formulation d'une plus grande quantité d'actif, pour maintenir son activité biologique. Il est donc nécessaire de disposer de formulations optimisant la quantité de rétinoïde vis-à-vis de son efficacité.

Pour ce faire, formuler un rétinoïde sous forme solubilisée est la solution la plus avantageuse en termes de coûts. En effet, solubiliser un rétinoïde dans une formulation permet d'en mettre une plus faible quantité que sous forme dispersée. Cependant, la tolérance de la formulation se trouve alors diminuée, l'actif pouvant être plus rapidement libéré ce qui facilite sa pénétration dans la peau.

L'art antérieur, et notamment le brevet US 5,650,171, décrit la formulation de rétinoïdes, en particulier la trétinoïne, avec des polyol pré-polymères à de fortes teneurs (>10%), ce qui complexifie la formulation.

Une autre solution consiste à inclure le rétinoïde dans des liposomes. Les liposomes comprennent des phospholipides qui enchâssent l'actif. Cependant, ces phospholipides sont difficiles à formuler, notamment à une échelle industrielle, et à stabiliser, notamment aux températures élevées comme par exemple à 40°C.

Enfin, l'art antérieur propose de solubiliser le rétinoïde, généralement hydrophobe, dans des solvants alcooliques, afin d'obtenir en général un gel hydroalcoolique. Les quantités de solvant utilisées ne sont alors pas adaptées aux pathologies pour lesquelles les rétinoïdes sont efficaces, à savoir les pathologies dermatologiques. La formulation Aberel® de Janssen-Cilag pour le traitement de l'acné (à base de trétinoïne 0.025% sous forme de gel et comprenant une grande quantité d'alcool) a notamment été retirée du marché car mal tolérée.

Il existe donc un besoin pour une formulation stable comprenant le rétinoïde sous forme solubilisée, faiblement irritante (donc bien tolérée), qui permette une libération contrôlée et une bonne pénétration de l'actif, qui soit acceptable du point de vue de ses propriétés organoleptiques, et qui soit facilement fabricable et industrialisable.

Le problème que se propose de résoudre ici la présente invention, est donc de concevoir une composition stable physiquement et chimiquement, comprenant au moins un rétinoïde, pour le traitement de pathologies dermatologiques, particulièrement l'acné, ledit rétinoïde étant sous forme solubilisée, la composition selon l'invention étant facilement industrialisable, et devant améliorer la tolérance du principe actif tout en présentant une facilité d'utilisation et une cosméticité acceptables pour une application sur toutes les zones du corps pouvant être touchées par la pathologie.

Selon la présente invention, le rétinoïde doit se présenter sous une forme solubilisée dans une composition stable. De nombreux rétinoïdes présentent souvent des difficultés de solubilisation. Les rétinoïdes selon l'invention, et notamment le rétinoïde préférentiellement utilisé, présentent une faible solubilité, ce qui limite l'incorporation de ceux-ci dans les véhicules classiques, et qui rend difficile l'obtention d'une composition stable. Par ailleurs, l'ajout d'un agent solubilisant dans les formulations topiques augmente souvent le pouvoir irritant des formules.

La présente invention porte donc sur la formulation d'oléosomes pouvant améliorer la tolérance cutanée de rétinoïdes dans le traitement de pathologies dermatologiques, notamment l'acné ; les ichtyoses, les états ichtyosiformes, l'hyperkératose palmoplantaire ou le psoriasis, préférentiellement l'acné.

La Demanderesse a donc, de façon surprenante, découvert que des compositions comprenant au moins un rétinoïde, sous forme solubilisée soit au sein d'oléosomes dispersés dans une phase aqueuse, soit directement solubilisé dans la phase aqueuse, ne nécessitant pas de polymère et/ou pas ou peu de solvant organique, garantissent la stabilité (chimique et physique) de l'actif et la bonne tolérance de la composition. Les compositions selon l'invention peuvent également favoriser la pénétration cutanée de l'actif, ce qui est utile dans le traitement d'affections dermatologiques,

notamment l 'acné. Dans un autre mode préféré selon l'invention, la demanderesse a découvert que la tolérance de l'actif était améliorée lorsque celui-ci était solubilisé dans la phase interne huileuse des oléosomes, tout en obtenant une libération contrôlée et une bonne pénétration de l'actif dans la peau.

Par oléosomes, on entend les globules liquides huileux unitairement enrobés d'une couche cristal liquide monolamellaire ou oligolamellaire obtenue à partir d'au moins un agent tensioactif lipophile, d'au moins un agent tensioactif hydrophile et d'au moins un tensioactif anionique distinct. Ces oléosomes sont dispersés dans la phase aqueuse (phase continue).

Par stabilité physique selon l'invention, on entend une composition dont les propriétés physiques, telles que les caractères organoleptiques, pH et viscosité, sont stables au cours du temps et à différentes conditions de température (par exemple 4°C, température ambiante et 40°C).

Par température ambiante, on entend une température comprise entre 15 et 25°C.

Par stabilité chimique selon l'invention, on entend une composition dans laquelle le principe actif est stable chimiquement au cours du temps et ce quelle que soit la condition de température : 4°C, température ambiante, 40°C.

La présente invention a donc notamment pour objet une composition, en particulier pharmaceutique, comprenant au moins un rétinoïde particulier, à savoir l'acide 3"-tert-butyl-4'-(2-hydroxy-éthoxy)-4"-pyrrolidin-1-yl-[1,1';3',1"]-terphényl-4-carboxylique, en tant qu'actif pharmaceutique, caractérisée en ce que :

- ladite composition est une émulsion huile-dans-eau formée de globules liquides huileux pourvus chacun d'un enrobage cristal liquide lamellaire et dispersés dans une phase aqueuse,
- ledit rétinoïde est solubilisé soit dans les globules liquides huileux, soit dans la phase aqueuse de l'émulsion huile-dans-eau,
- chaque globule liquide huileux est unitairement enrobé d'une couche monolamellaire ou oligolamellaire obtenue à partir d'au moins un agent tensioactif lipophile, d'au moins un agent tensioactif hydrophile et d'au moins un tensioactif anionique distinct.

Dans un mode préféré, la présente invention a pour objet une composition, notamment pharmaceutique, comprenant :

- une émulsion huile-dans-eau formée de globules liquides huileux pourvus chacun d'un enrobage cristal liquide lamellaire et dispersés dans une phase aqueuse,
- chaque globule liquide huileux est unitairement enrobé d'une couche monolamellaire ou oligolamellaire obtenue à partir d'au moins un agent tensioactif lipophile, d'au moins un agent tensioactif hydrophile et d'au moins un tensioactif anionique distinct, caractérisée en ce que ladite composition comprend l'acide 3"-tert-butyl-4'-(2-hydroxy-éthoxy)-4"-pyrrolidin-1-yl-[1,1';3',1"]-terphenyl-4-carboxylique solubilisé dans les globules liquides huileux.

Dans un autre mode selon l'invention, l'acide 3"-tert-butyl-4'-(2-hydroxy-éthoxy)-4"-pyrrolidin-1-yl-[1,1';3',1"]-terphenyl-4-carboxylique est en partie solubilisé dans les globules liquides huileux et en partie solubilisé dans la phase externe aqueuse de la composition.

La présente invention a également pour objet l'utilisation d'une composition comprenant au moins un rétinoïde :

- ladite composition étant une émulsion huile-dans-eau comprenant des globules liquides huileux pourvus chacun d'un enrobage cristal liquide lamellaire et dispersés dans une phase aqueuse,
- le rétinoïde étant solubilisé soit dans les globules liquides huileux, soit dans la phase aqueuse de l'émulsion huile-dans-eau,
- chaque globule liquide huileux étant unitairement enrobé d'une couche monolamellaire ou oligolamellaire obtenue à partir d'au moins un agent tensioactif lipophile, d'au moins un agent tensioactif hydrophile et d'au moins un tensioactif anionique distinct, et les globules liquides huileux enrobés ayant un diamètre moyen inférieur à 800 nm,

pour améliorer la tolérance dudit rétinoïde.

De préférence, le rétinoïde mis en oeuvre au cours de cette utilisation est l'acide 3"-tert-butyl-4'-(2-hydroxy-éthoxy)-4"-pyrrolidin-1-yl-[1,1';3',1"]-terphenyl-4-carboxylique.

Un autre objet de la présente invention est l'utilisation de la composition précédemment définie pour améliorer la tolérance du rétinoïde lorsque celui-ci est solubilisé dans la phase interne huileuse de la composition, tout en contrôlant la libération et la pénétration de l'actif.

De préférence, le rétinoïde utilisé est l'acide 3"-tert-butyl-4'-(2-hydroxy-éthoxy)-4"-pyrrolidin-1-yl-[1,1';3',1"]-terphényl-4-carboxylique.

Selon un autre mode, l'invention concerne l'utilisation d'une composition telle que définie précédemment pour améliorer la pénétration cutanée du rétinoïde, ladite composition ne contenant pas de propénétrant.

De préférence, le rétinoïde utilisé est l'acide 3"-tert-butyl-4'-(2-hydroxy-éthoxy)-4"-pyrrolidin-1-yl-[1,1';3',1"]-terphényl-4-carboxylique.

L'invention concerne également l'utilisation d'une composition telle que définie précédemment pour obtenir une libération contrôlée du rétinoïde, caractérisé en ce qu'il est solubilisé dans les globules liquides huileux.

De préférence, le rétinoïde utilisé est l'acide 3"-tert-butyl-4'-(2-hydroxy-ethoxy)-4"-pyrrolidin-1-yl-[1,1';3',1"]-terphenyl-4-carboxylique.

Les rétinoïdes pouvant être utilisés dans le cadre de l'invention comprennent notamment l'acide tout-trans rétinoïque ou trétinoïne, l'acide 13-cis-rétinoïque ou isotrétinoïne, l'acitrétine, l'acide arotinoïque, le rétinol, l'adapalène, le tazarotène, le rétinaldéhyde, l'étrétinate et, les composés protégés dans la demande de brevet WO2006/066978 tel que l'acide 3"-tert-butyl-4'-(2-hydroxy-ethoxy)-4"-pyrrolidin-1-yl-[1,1';3',1"]-terphenyl-4-carboxylique, les composés de la demande de brevet FR0512367 dont l'acide 2-hydroxy-4-[3-hydroxy-3-(5,6,7,8-tétrahydro-5,5,8,8-tétraméthyl-2-naphtyl)-1-propynyl]benzoique ou l'un de ses énantiomères, les composés de la demande de brevet WO 05/56516 dont l'acide 4'-(4-isopropylamino-butoxy)-3'-(5,5,8,8-tetramethyl-5,6,7,8-tetrahydro-naphthalen-2-yl)-biphenyl-4-carboxylique, les composés de la demande de brevet PCT/EP04/014809 dont l'acide 4-{3-hydroxy-3-[4-(2-éthoxy-ethoxy)-5,5,8,8-tetramethyl-5,6,7,8-tetrahydro-naphthalen-2-yl]-prop-1-ynyl}-benzoïque, les composés de la demande de brevet FR 2 861 069 dont l'acide 4-[2-(3-tert-butyl-4-diethylamino-phenyl)-2-hydroxyimino-ethoxy]-2-hydroxy-benzoïque.

L'acide 3"-tert-butyl-4'-(2-hydroxy-éthoxy)-4"-pyrrolidin-1-yl-[1,1';3',1"]-terphenyl-4-carboxylique tel que protégé dans la demande WO2006/066978 est particulièrement préféré, et sera appelé « Composé A » dans la suite de la présente demande.

La composition selon l'invention comprend entre 0.00001 et 1% d'au moins un rétinoïde en poids par rapport au poids total de la composition, de préférence de 0.0001 à 0.5% et de manière préférentielle, la composition selon l'invention contient de 0.001% à 0.05% d'un rétinoïde en poids par rapport au poids total de la composition. Dans un mode préféré selon l'invention, la composition comprend entre 0.001 et 0.05% d'acide 3"-tert-butyl-4'-(2-hydroxy-ethoxy)-4"-pyrrolidin-1-yl-[1,1';3',1"]-terphenyl-4-carboxylique, plus particulièrement entre 0.003 et 0.03 % en poids par rapport au poids total de la composition.

Selon la présente invention, le rétinoïde est solubilisé soit dans les globules liquides huileux, soit dans la phase aqueuse de l'émulsion huile-dans-eau. De préférence, le rétinoïde est solubilisé dans les globules liquides huileux (ces globules liquides huileux sont aussi appelés aussi « phase interne » ou « phase interne huileuse » dans la présente invention). En effet, la demanderesse a découvert de manière surprenante que l'internalisation de l'actif dans les globules liquides huileux de la composition assure une meilleure tolérance de la composition, tout en conférant une libération contrôlée et une bonne pénétration de l'actif, comparable aux formulations de référence dans lesquelles l'actif est en phase aqueuse externe.

De plus, lorsque le rétinoïde est solubilisé dans la phase interne de l'invention, il est possible de limiter ou de s'affranchir de l'utilisation de propénétrant. En effet, bien que souvent indispensable pour améliorer la pénétration des actifs pharmaceutiques et donc leur efficacité, les propénétrants peuvent présenter l'inconvénient d'être irritants. Aussi, selon un mode de réalisation préféré, lorsque le rétinoïde est solubilisé dans les globules liquides huileux, la composition ne contient substantiellement pas de propénétrant, de préférence elle est exempte de tout propénétrant.

Par composition ne contenant substantiellement pas de propénétrant, on entend une composition contenant moins de 0.5%, de préférence moins de 0.3%, de préférence moins de 0.1% en poids de propénétrant.

Par propénétrant, on entend les alcools comme l'éthanol ; les glycols comme par exemple le propylène glycol ; les éthers de glycols comme par exemple le PPG-15 stéaryl éther ; la N-méthyl-2-pyrrolidone ; le diméthylsulfoxyde ou encore le diméthylisosorbide.

La composition selon l'invention peut être incorporée dans un véhicule pharmaceutiquement acceptable, tel un gel, une solution ou une émulsion comme une crème ou une lotion, une mousse, pouvant être pulvérisable ou non, pressurisée ou non.

Selon la présente invention, la composition comprend des oléosomes et non des nanosphères lipidiques. En effet, les oléosomes sont des globules liquides huileux enrobés par une enveloppe non polymérique (i.e. formée à partir d'au moins un agent tensioactif lipophile, d'au moins un agent tensioactif hydrophile et d'au moins un tensioactif anionique distinct), par opposition à des nanosphères lipidiques qui sont des particules matricielles, i.e. dont la totalité de la masse est solide à température ambiante.

Les oléosomes (globules liquides huileux) selon la présente invention présentent une taille moyenne inférieure à 800nm, de préférence, inférieur à 500nm. Le diamètre moyen des oléosomes et la distribution granulométrique peuvent être déterminés par DLS (Dynamic Light Scattering) au moyen d'un granulomètre de type Zetasizer Nano ZS (Malvern Instruments), comme cela est expliqué dans les exemples.

La distribution granulométrique des oléosomes peut également être contrôlée en cours de fabrication, lors de la préparation de la phase « oléosomes », afin de s'assurer de la finesse de l'émulsion, par prélèvement d'un échantillon.

Les oléosomes sont donc constitués de globules huileux pourvus chacun d'un enrobage cristal liquide lamellaire, l'enrobage étant une couche monolamellaire ou oligolamellaire obtenue à partir d'au moins un agent tensioactif lipophile,

d'au moins un agent tensioactif hydrophile et d'au moins un tensioactif anionique distinct ; ils sont dispersés dans la phase aqueuse.

Par couche oligolamellaire, on entend une couche comprenant de 2 à 5 lamelles lipidiques, comme cela est décrit dans le brevet EP 0 641 557.

De préférence, l'agent tensioactif lipophile utilisé pour la formation des oléosomes présente une HLB comprise entre 2 et 5.

A titre d'agent tensioactif lipophile présentant une HLB comprise entre 2 et 5 utilisable selon l'invention, on citera les esters de sorbitane, tels que le monostéarate de sorbitane (HLB = 4.7) vendu sous le nom de Span® 60 par la société Croda, les esters de glycérol tel que le monostéarate de glycerol vendu sous le nom de Cutina® GMSVPH (HLB=3.8) par la société BASF, les sucroesters de basse HLB tel que le sucrose dilaurate vendu sous le nom de Surfhope® C-1205 (HLB=5) ou le sucrose tristéarate vendu sous le nom de Surfhope® C-1803 (HLB=3) par la société Gattefossé, ou encore le stéaryl éther polyoxyéthyléné comportant 2 motifs oxyéthylène (2 OE).

De préférence, l'agent tensioactif lipophile présentant une HLB comprise entre 2 et 5 est choisi parmi le distéarate de sucrose, le distéarate de diglycéryle, le tristéarate de tétraglycéryle, le décastéarate de décaglycéryle, le monostéarate de diglycérol, le monostéarate de sorbitane, le tristéarate de sorbitane, le monostéarate de 15 diéthylène glycol, l'ester de glycérol et d'acides palmitique et stéarique, le monostéarate polyoxyéthyléné comportant 2 motifs oxyéthylène (2 OE), le stéaryl éther polyoxyéthyléné comportant 2 motifs oxyéthylène (2 OE) soit le Steareth-2, le mono- et dibéhénate de glycéryle et le tétrastéarate de pentaérythritol.

De préférence, l'agent tensioactif hydrophile utilisé pour la formation des oléosomes présente une HLB comprise entre 8 et 12.

On peut citer, à titre d'exemple non limitatif d'agent tensioactif hydrophile présentant une HLB entre 8 et 12, les esters de polyoxyéthylène glycol tel que le glycéryl stéarate and PEG 100 stéarate vendu sous le nom de Arlacel® 165 FL (HLB=11) par la société Croda , le PEG 6 Stéarate et PEG 32 stéarate vendu sous le nom de Tefose® 1500 (HLB= 10) par la société Gateffossé, les esters de sorbitane polyoxyéthyléné et les sucroesters de haut HLB tel que le sucrose stéarate vendu sous le nom de Surfhope® C-1811 (HLB=11) par la société Gattefossé, ou encore le stéaryl éther polyoxyéthyléné comportant 10 motifs oxyéthylène.

De préférence, l'agent tensioactif hydrophile présentant une HLB comprise entre 8 et 12 est choisi parmi le monostéarate de sorbitane polyoxyéthyléné 4 OE, le tristéarate de sorbitane polyoxyéthyléné 20 OE, le monostéarate polyoxyéthyléné 8 OE, le monostéarate d'hexaglycéryle, le monostéarate polyoxyéthyléné 10 OE, le stéaryl éther polyoxyéthyléné 10 OE soit le steareth-10, le distéarate polyoxyéthyléné 12 OE et le distéarate de méthylglucose polyoxyéthyléné 20 OE.

Par tensioactif anionique distinct, on entend un tensioactif anionique différent de l'agent tensioactif lipophile et de l'agent tensioactif hydrophile formant l'enrobage des oléosomes.

De préférence, le tensioactif anionique distinct est un acide gras ou un ester d'acide gras, ledit acide gras comprenant au moins une chaîne grasse saturée ayant plus de 12 atomes de carbone, de préférence entre 16 et 22.

Le tensioactif anionique distinct peut également être un lipide amphiphile ionique. Le lipide amphiphile ionique est de préférence choisi parmi le groupe comprenant les lipides anioniques neutralisés, les lipides amphotères et les dérivés alkylsulfoniques.

Les lipides anioniques neutralisés sont choisis en particulier parmi :

- les sels alcalins du dicétylphosphate, et en particulier les sels de sodium et de potassium ;
- les sels alcalins du dimyristylphosphate, et en particulier les sels de sodium et de potassium ;
- les sels alcalins du cholestérol sulfate, et en particulier le sel de sodium ;
- les sels alcalins du cholestérol phosphate, et en particulier le sel de sodium ;
- les sels monosodique et disodique des acides acylglutamiques, et en particulier les sels monosodique et disodique de l'acide N-stéaroyl glutamique ou le sel de sodium de l'acide phosphatidique, comme le stéaroyl glutamate de sodium commercialisé sous la dénomination Eumulgin SG par Cognis.

Les lipides amphotères sont choisis en particulier parmi les phospholipides et notamment la phosphatidyléthanolamine de soja pure.

Les dérivés alkylsulfoniques sont avantageusement les composés de formule :

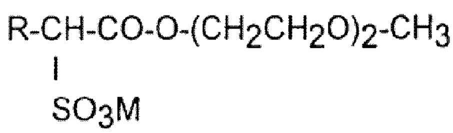

dans laquelle R représente les radicaux $C_{16}H_{33}$ et $C_{18}H_{37}$ pris en mélange ou séparément et M est un métal alcalin, de préférence le sodium.

Plus préférentiellement, le tensioactif anionique distinct est choisi parmi l'acide stéarique, l'acide palmitique, l'acide arachidique et l'acide béhénique.

Dans le cas de l'utilisation d'un acide gras et afin de permettre au système lamellaire de se former, le pH de la composition selon l'invention est important et doit être supérieur à 7 (basique). En effet, à pH supérieur à 7, les acides gras sont anioniques.

L'agent basique permettant la neutralisation de l'acide gras présent dans les oléosomes peut être la triéthanolamine, la soude, la lysine ou bien encore l'arginine.

De préférence, les tensioactifs sont mélangés dans les proportions suivantes pour former les oléosomes :

- ◦ Tensioactif lipophile (HLB comprise entre 2 et 5) : 45-50% en poids du mélange ;
- ◦ Tensioactif hydrophile (HLB comprise entre 8 et 12) : 30-35% en poids du mélange ; et
- ◦ Tensioactif anionique distinct : 20-25% en poids du mélange.

De préférence, la quantité totale de tensioactifs présents dans une composition obtenue par le procédé selon l'invention est comprise entre 3 et 4% en poids total de la composition.

Un oléosome selon l'invention est constitué d'un enrobage et d'un globule liquide huileux dans lequel le rétinoïde peut être solubilisé. De préférence, le rétinoïde, et plus particulièrement l'acide 3"-tert-butyl-4'-(2-hydroxy-ethoxy)-4"-pyrrolidin-1-yl-[1,1';3',1"]-terphenyl-4-carboxylique, se trouve solubilisé dans les globules liquides huileux.

La composition des globules liquides huileux est essentielle pour la stabilité du rétinoïde. Ces globules liquides huileux doivent bien entendu être compatibles avec le rétinoïde à solubiliser, et doivent être solubilisants du rétinoïde.

De préférence, les globules liquides huileux comprennent au moins un solvant huileux choisi parmi le phénoxyéthanol, les éthers de glycols, les acides gras polyéthoxylés, les triglycérides et huiles en contenant et les esters d'acides gras.

Par solvant huileux, on entend toute matière non miscible à l'eau d'origine naturelle, animale ou synthétique. Le solvant huileux est un solvant dans lequel l'actif est solubilisé et stable chimiquement. Il peut être présent à une concentration allant de 0.01 à 20% en poids.

Parmi les triglycérides et huiles en contenant, on peut citer à titre non limitatif, les triglycérides d'acide octanoïque ou encore les triglycérides des acides caprylique/caprique comme ceux vendus par la société Stearineries Dubois ou ceux vendus sous la dénomination Miglyol® 810, 812, et 818 par la société Sasol.

Parmi les éthers de glycols on peut citer à titre non limitatif les éthers de polypropylène glycol comme le PPG-15 stéaryl éther vendu sous le nom d'Arlamol® PS15E par la société Croda. Parmi les esters d'acide gras on peut citer à titre non limitatif le diisopropyl adipate vendu sous le nom de Crodamol® DA par la société Croda, le cétéaryl isononanoate vendu sous le nom de Kollicream® CI par la société BASF.

Dans le cas où l'actif de la composition selon l'invention est le Composé A, le solvant huileux peut être, par exemple, le phénoxyéthanol vendu sous le nom de Phenoxetol® par Clariant, présent à une concentration allant de 0.2 à 5%, et préférentiellement de 0.5 à 2% en poids.

En sus de ce(s) solvant(s) huileux, les globules liquides huileux peuvent également comprendre un ou plusieurs corps gras liquides ou semi-liquides à température ambiante non solubilisants du rétinoïde. Ces composés sont notamment des huiles minérales, des huiles végétales naturelles ou synthétiques, des huiles animales ou des huiles de silicone.

La phase grasse de l'invention peut également comprendre :

- Une ou plusieurs huiles minérales, comme les huiles de paraffine de différentes viscosités comme le Marcol® 152, le Marcol® 52 ou le Primol® 352 vendu par Univar
- Une ou plusieurs huiles végétales parmi lesquelles on peut citer l'huile d'amande douce, l'huile de palme, l'huile de soja, l'huile de sésame, l'huile de tournesol, l'huile de ricin hydrogénée, l'huile de coprah,
- Une ou plusieurs huiles synthétiques parmi lesquelles on peut citer l'Apricot Kernel Oil PEG-6 ester (Labrafil® M1944CS), le propylène glycol laurate (Lauroglycol® FCC), le propylène glycol monocaprylate (Capryol® 90) fournis par Gattéfossé, des esters tel que le cétéaryl isononanoate comme le produit vendu sous le nom de Kollicream® CI par la société BASF France, le palmitate d'isopropyle comme le produit vendu sous le nom de Crodamol® IPP par la société Croda,
- Une ou plusieurs huiles animales parmi lesquelles on peut citer la lanoline, le squalène, l'huile de poisson, l'huile de vison avec comme dérivé le squalane vendu sous le nom Cosbiol® par la société Laserson.
- Une ou plusieurs huiles de silicone améliorant les propriétés de la formule à l'application, comme la Cyclomethicone (St-Cyclomethicone® 5NF) ou la Dimethicone (Q7 9120 Silicon Fluid® de viscosité de 20 cSt à 12500 cSt de Dow Corning),
- un ou plusieurs épaississants de phase grasse de type alcool gras comme l'alcool cétylique (Crodacol® C70 fourni

par Croda/ Lanette 16 fourni par BASF mais aussi Kolliwax® CA fourni par BASF), l'alcool cétearylique (Crodacol® 1618 fourni par Croda, Tego Alkanol® 1618 fourni par Evonik mais aussi le Kolliwax® CSA Pharma fourni par BASF), l'alcool stéarylique (Crodacol® S95 fourni par Croda, Kolliwax® SA fourni par BASF mais aussi Tego Alkanol® 18 fourni par Evonik) mais aussi l'alcool behenique (Lanette® 22 fourni par BASF, Nacol® 22-98 fourni par Sasol mais aussi Behenyl Alcohol 65 80 fourni par Nikko Chems) ou de type cire de Carnauba fourni par Baerlocher mais aussi la cire d'abeille vendu sous le nom de Cerabeil Blanchie DAB® fourni par Univar, le tribéhénate de glycéryle tel que le Compritol® 888 fourni par Gattéfossé. Dans ce cas, l'homme du métier adaptera la température de chauffage de la préparation en fonction de la présence ou non de ces solides.

D'autres huiles ou corps gras peuvent être ajoutés à la phase grasse de la composition de manière variée par l'homme du métier afin de préparer une composition ayant les propriétés souhaitées, par exemple en consistance ou en texture.

De préférence, la composition selon l'invention présente un rapport entre la quantité totale de tensioactifs et la quantité totale de phase huileuse constituant les globules liquides huileux compris entre 10% et 25%. Avec un tel rapport, les oléosomes présentent une taille acceptable, avec un enrobage cristal liquide lamellaire autour des gouttelettes d'huile mono- ou oligolamellaire.

Les oléosomes sont dispersés dans une phase aqueuse, qui est la phase continue. Cette dernière comprend de l'eau. Cette eau peut être de l'eau déminéralisée, une eau florale telle que l'eau de bleuet, ou une eau thermale ou minérale naturelle, par exemple choisie parmi l'eau de Vittel, les eaux du bassin de Vichy, l'eau d'Uriage, l'eau de la Roche Posay, l'eau de la Bourboule, l'eau d'Enghien-les-Bains, l'eau de Saint Gervais-les-Bains, l'eau de Néris-les-Bains, l'eau d'Allevard-les-Bains, l'eau de Digne, l'eau de Maizières, l'eau de Neyrac-les-Bains, l'eau de Lons-le-Saunier, les Eaux Bonnes, l'eau de Rochefort, l'eau de Saint Christau, l'eau des Fumades et l'eau de Tercis-les-bains, l'eau d'Avène ou l'eau d'Aix les Bains.

L'eau peut être présente à une teneur comprise entre 25 et 90 % en poids par rapport au poids total de la composition, de préférence comprise entre 50 et 90 % en poids.

La phase aqueuse peut contenir de manière non limitative un ou plusieurs polyols, différents types d'additifs.

Selon un mode préféré, la composition selon l'invention présente un rapport entre la phase aqueuse et la phase grasse (globules liquides huileux enrobés) suivant:

% Phase grasse / (% phase grasse + % phase aqueuse) compris entre 0.40 et 0.55, de préférence comprise entre 0.45 et 0.50, de préférence égal à 0.48.

La composition selon l'invention peut être incorporée au sein d'un véhicule pharmaceutiquement acceptable, tel un gel, une solution ou une émulsion comme une crème ou une lotion, une mousse.

Lorsque le véhicule pharmaceutiquement acceptable est un gel, la composition selon l'invention est dispersée dans une phase hydrophile qui comprend au moins un agent gélifiant. Cet agent gélifiant peut être un dérivé cellulosique choisi parmi les gélifiants cellulosiques semi-synthétiques, tels que la méthylcellulose, l'éthylcellulose, l'hydroxypropylméthylcellulose commercialisés par la société Colorcon sous le nom de Methocel® (par exemple : Methocel® E4M), l'hydroxyéthylcellulose commercialisés par la société Ashland sous le nom de Natrosol® (par exemple : Natrosol® 250 HHX), la carboxyméthylcellulose, l'hydroxyméthylcellulose, et l'hydroxypropylcellulose, pris seuls ou en mélange.. L'agent gélifiant peut également être choisi parmi les gommes naturelles comme la gomme de tragacanthe, la gomme de guar, la gomme d'acacia, la gomme arabique, la gomme xanthane, l'amidon et ses dérivés, les copolymères d'acide polyacrylique comme par exemple les carbomers commercialisés par la société Lubrizol (i.e.Carbomer 980) et de methylmethacrylate, les carboxyvinyl polymères, les polyvinyl pyrrolidones et leurs dérivés, les polyvinyl alcools, les biopolymères tels que l'alginate de sodium, la pectine, la dextrine, le chitosan, le hyaluronate de sodium et leurs dérivés pris seuls ou en mélange, L'agent gélifiant peut également être choisi parmi le composé Sepigel® 305 constitué d'un mélange polyacrylamide / isoparaffine en C13-C14/ laureth-7, ou le Simulgel® 600PHA ou Sepineo® P600, à savoir le sodium acryloyldiméthyltaurate copolymère/ isohexadecane/polysorbate 80, ces deux produits étant commercialisés par la société Seppic. De préférence, on utilise le Simulgel® 600PHA.

L'agent gélifiant est utilisé notamment à une concentration comprise entre 0,1 et 10% en poids, de préférence entre 0,1 et 4% en poids par rapport au poids total de la composition.

Lorsque le véhicule pharmaceutiquement acceptable est une solution, la composition selon l'invention est dispersée au sein d'un véhicule composé d'une phase aqueuse (telle que définie précédemment dans la présente invention).

Lorsque le véhicule pharmaceutiquement acceptable est une crème ou une lotion, la composition selon l'invention est dispersée au sein d'un véhicule composé d'une phase aqueuse et d'une phase grasse comprenant ou non au moins un surfactant ou émulsionnant.

La composition selon l'invention pourra en outre contenir des additifs ou combinaisons d'additifs, tels que :

- des agents co-tensioactifs comme les alcools gras ;

- des agents stabilisants ;
- des agents humectants ;
- des agents régulateurs d'humidité ;
- des agents régulateurs de pH ;
- des agents modificateurs de pression osmotique ;
- des agents chélatants ;
- des agents conservateurs;
- des filtres UV-A et UV-B ;
- et des antioxydants.

Bien entendu, l'homme du métier veillera à choisir le ou les éventuels composés à ajouter à ces compositions de telle manière que les propriétés avantageuses attachées intrinsèquement à la présente invention ne soient pas ou substantiellement pas altérées par l'addition envisagée.

Ces additifs peuvent être présents dans la composition de 0 à 40% en poids par rapport au poids total de la composition.

La composition pharmaceutique utilisable selon l'invention est destinée au traitement de la peau et peut être administrée par voie topique, parentérale ou orale. De préférence, la composition est administrée par voie topique. Par voie topique, on entend une application sur la peau ou les muqueuses, ainsi que sur les cheveux ou le cuir chevelu.

Par voie orale, la composition pharmaceutique peut se présenter sous forme liquide, ou pâteuse, et plus particulièrement sous formes de gélules, de dragées, ou de sirops.

Par voie parentérale, la composition peut se présenter sous forme de suspensions pour perfusion ou pour injection.

Par voie topique, la composition peut se présenter sous forme liquide, ou pâteuse, et plus particulièrement sous forme de crèmes, de laits, de pommades, de tampons imbibés, de syndets, de lingettes, de gels, de sprays, de mousses, de lotions, de sticks, de shampoings, ou de bases lavantes.

La composition sous forme d'émulsion huile-dans-eau comprenant les globules liquides huileux selon la présente invention peut être obtenue par simple mélange de deux phases, qui sont :

- Une phase aqueuse chauffée, à une température d'au plus 75°C, de préférence comprise entre 65°C et 75°C,
- Une phase huileuse chauffée, à une température d'au plus 75°C, de préférence comprise entre 65°C et 75°C,, et contenant le mélange de tensioactifs (i.e. tensioactif lipophile, tensioactif hydrophile et tensioactif anionique distinct).

L'émulsification est obtenue soit à l'aide d'un Homogénéisateur Haute Pression (HHP), soit par simple mélange des deux phases chauffées à l'aide d'une turbine d'agitation. Dans ce dernier cas, le rapport entre la phase aqueuse et la phase grasse est de préférence comme suit :

$$\% \text{ Phase grasse} / (\% \text{ phase grasse} + \% \text{ phase aqueuse}) = 0.48.$$

La composition selon l'invention est utilisable comme médicament.

En particulier, l'invention a également pour objet une composition pour son utilisation dans le traitement des affections dermatologiques, notamment humaines telles que définies ci-après :

1) les affections dermatologiques liées à un désordre de la kératinisation portant sur la différenciation et sur la prolifération cellulaire notamment pour traiter les acnés vulgaires, comédoniennes, polymorphes, rosacées, les acnés nodulokystiques, conglobata, les acnés séniles, les acnés secondaires telles que l'acné solaire, médicamenteuse ou professionnelle ;
2) les troubles de la kératinisation, notamment les ichtyoses, les états ichtyosiformes, l'ichtyose lamellaire, la maladie de Darrier, les kératodermies palmoplantaires, les leucoplasies, le pityriasis rubra pilaire et les états leucoplasiformes, le lichen cutané ou muqueux (buccal) ;
3) les affections dermatologiques avec une composante immuno-allergique inflammatoire, avec ou sans trouble de la prolifération cellulaire, et notamment toutes les formes de psoriasis, qu'il soit cutané, muqueux ou unguéal, et même le rhumatisme psoriasique, ou encore la dermatite atopique et les différentes formes d'eczéma;
4) les désordres cutanés dus à une exposition aux rayonnements U.V. ainsi que pour réparer ou lutter contre le vieillissement de la peau, qu'il soit photo-induit ou chronologique ou pour réduire les pigmentations et les kératoses actiniques, ou toutes pathologies associées au vieillissement chronologique ou actinique, telle la xérose, les pigmentations et les rides ;
5) toute condition liée à des proliférations dermiques ou épidermiques bénignes, qu'elles soient ou non d'origine

virale telles que verrues vulgaires, les verrues planes , le molluscum contagiosum et l'épidermodysplasie verruciforme, les papillomatoses orales ou florides,;
6) les désordres dermatologiques tels que les dermatoses immunes comme le lupus érythémateux, les maladies immunes bulleuses et les maladies du collagène, telle la sclérodermie ;
7) les stigmates de l'atrophie épidermique et/ou dermique induite par les corticostéroïdes locaux ou systémiques, ou toute autre forme d'atrophie cutanée,
8) les troubles de la cicatrisation, ou pour prévenir ou pour réparer les vergetures, ou encore pour favoriser la cicatrisation,
9) dans le traitement de toute affection d'origine fongique au niveau cutané tel que le tinea pedis et le tinea versicolor,
10) les désordres de la pigmentation, tel l'hyperpigmentation, le mélasma, l'hypopigmentation ou le vitiligo ;
11) les états cancéreux ou précancéreux, cutanés ou muqueux comme les kératoses actiniques, la maladie de Bowen, les carcinomes in-situ, le kératoacanthome et les cancers cutanés comme le carcinome basocellulaire (BCC), le carcinome spinocellulaire (SCC) et les lymphomes cutanés tek que le lymphome T.

De préférence, l'invention porte sur une composition pour son utilisation dans le traitement de l'acné, les ichtyoses, les états ichtyosiformes, l'hyperkératose palmoplantaire ou le psoriasis.

La composition selon l'invention convient en effet pour une utilisation dans le traitement d'affections dermatologiques, notamment humaines, et de préférence pour le traitement de l'acné.

La présente invention concerne également l'utilisation d'une composition comprenant au moins un rétinoïde pour améliorer la tolérance du rétinoide, ladite composition étant une émulsion huile-dans-eau comprenant des globules liquides huileux pourvus chacun d'un enrobage cristal liquide lamellaire et dispersés dans une phase aqueuse, le rétinoïde étant solubilisé soit dans les globules liquides huileux, soit dans la phase aqueuse de l'émulsion huile-dans-eau, chaque globule liquide huileux étant unitairement enrobé d'une couche monolamellaire ou oligolamellaire obtenue à partir d'au moins un agent tensioactif lipophile, d'au moins un agent tensioactif hydrophile et d'au moins un tensioactif anionique distinct, et les globules liquides huileux enrobés ayant un diamètre moyen inférieur à 800 nm,

L'utilisation de la composition selon l'invention est également destinée à améliorer la tolérance du rétinoïde lorsque celui-ci est solubilisé dans la phase interne huileuse de la composition, tout en contrôlant la libération et la pénétration de l'actif.

Dans un autre mode, l'invention concerne donc l'utilisation d'une composition selon l'invention pour améliorer la pénétration cutanée du rétinoïde, la dite composition ne contenant pas de propénétrant.

L'invention concerne également l'utilisation d'une composition selon l'invention pour obtenir une libération contrôlée du rétinoïde, caractérisé en ce qu'il est solubilisé dans les globules liquides huileux.

Il va être maintenant donné, à titre d'illustration et sans aucun caractère limitatif, diverses formulations de compositions selon l'invention.

Dans les différents exemples listés par la suite :

- la phase A désigne la phase huileuse constitutive du globule liquide huileux,
- la phase B désigne la phase aqueuse,
- la phase C désigne la phase constituée du ou des gélifiants et autres additifs, non incorporés dans les phases huileuses (A) ou aqueuses (B).

**<u>Exemple 1 : Données de solubilité du composé A dans différents solvants huileux et aqueux</u>**

L'objectif de cette étude de préformulation est d'identifier des solvants lipophiles et hydrophiles dans lesquels le composé A est soluble, et dans lesquels il est stable chimiquement.

La stabilité de l'actif a été évaluée par chromatographie en phase liquide couplée à un détecteur UV (HPLC-UV).

| Nom INCI (nom commercial) | Solubilité maximale (%m/m) | Stabilité* |
|---|---|---|
| Phénoxyéthanol | 1.957 | 6 mois TA / 40°C |
| Ethanol absolu | 0.92 | 3 mois TA / 40°C |
| Propylène glycol | 0.11 | 3 mois TA / 40°C |
| Propylène glycol / Ethanol absolu (20/80) | 1.08 | 6 mois TA / 40°C |
| PPG-15 stearyl éther (Arlamol E PSE15 | 0.292 | 6 mois TA / 40°C |

(suite)

| Nom INCI (nom commercial) | Solubilité maximale (%m/m) | Stabilité* |
|---|---|---|
| Triglycérides d'acides caprylique / caprique (Miglyol 812N) | 0.019 | 6 mois TA / 40°C |
| * : TA Température ambiante | | |

Cette étude nous permet par ailleurs de confirmer que l'actif est idéalement solubilisé dans des composés de type alcool ou glycol et est faiblement soluble dans les solvants lipophiles comme le Mygliol 812.

En fonction de la phase d'introduction du composé A, les solvants suivants seront donc utilisés :

- l'éthanol et le propylène glycol en mélange, dans les compositions comprenant le composé A en phase aqueuse,
- le PPG-15 stéaryl éther, le Miglyol et le phénoxyéthanol en mélange, dans les compositions comprenant le composé A au sein des globules liquides huileux.

**Exemple 2: Compositions selon l'invention comprenant le composé A dans les globules liquides huileux**

i) Compositions 1 et 1' :

La composition 1 comprend les ingrédients suivants :

| Phase | Ingrédients | Teneur (%m/m) |
|---|---|---|
| A | Steareth-10 | 1.10 |
| A | Acide stéarique | 0.70 |
| A | Alcool stéarylique | 0.25 |
| A | Steareth-2 | 1.60 |
| A | Triglycérides d'acides caprique / caprylique | 15.00 |
| A | Phénoxyéthanol | 1.00 |
| A | DL-alpha tocophérol | 0.20 |
| A | Composé A | 0.01 |
| B | Disodium édétate | 0.10 |
| B | Glycérine | 5.00 |
| B | Eau purifiée | 43.75 |
| B | Triéthanolamine | 0.20 |
| C | Carbomer 980 | 0.50 |
| C | Eau purifiée | 30.00 |
| C | Triéthanolamine | 0.60 |

La composition 1' comprend les ingrédients suivants :

| Phase | Ingrédients | Teneur (%m/m) |
|---|---|---|
| A | Steareth-10 | 1.25 |
| A | Acide stéarique | 0.85 |
| A | Alcool stéarylique | 0.25 |
| A | Steareth-2 | 1.80 |
| A | Triglycérides d'acides caprique / caprylique | 15.50 |

(suite)

| Phase | Ingrédients | Teneur (%m/m) |
|---|---|---|
| A | Cyclopentasiloxane | 4.00 |
| A | Phénoxyéthanol | 1.00 |
| A | DL-alpha tocophérol | 0.20 |
| A | Composé A | 0.01 |
| B | Disodium édétate | 0.10 |
| B | Glycérine | 5.00 |
| B | Méthyl parabène | 0.20 |
| B | Eau purifiée | 21.80 |
| B | Triéthanolamine | 0.20 |
| C | Acrylamide / sodium acryloyldiméthyl taurate copolymère & isohexadécane & polysorbate 80 | 4.00 |
| C | Eau purifiée | 43.85 |

Il peut être noté que les compositions 1 et 1' ne comprennent aucun ingrédient propénétrant.

ii) Procédé de fabrication des compositions 1 et 1' :

**A/Préparation de l'émulsion**

- Dans un bécher formulaire de taille adaptée taré, peser tous les éléments de la phase grasse (A) et les chauffer à 70°C, sous agitation magnétique, jusqu'à l'obtention d'un mélange homogène.
- Dans un bécher formulaire annexe, peser tous les éléments de la phase aqueuse (B) dans une partie de l'eau et les chauffer à 70°C, sous agitation magnétique, jusqu'à l'obtention d'un mélange homogène.
- Verser rapidement la phase aqueuse (B) sur la phase grasse (A) sous agitation forte (Rotor-Stator de type Polytron® - Vitesse maximale 13000 rpm). Maintenir l'agitation pendant 40 minutes.

**B/Dilution de l'émulsion**

- Arrêter l'agitation au Polytron® et mettre le bécher contenant l'émulsion dans un bain-marie froid (glace pilée si possible), pour favoriser un refroidissement rapide.
- Mettre alors l'émulsion sous agitation lente (Pâle défloculeuse, Rayneri - 200 rpm).
- Ajouter progressivement le restant de l'eau de la phase aqueuse (B) préalablement refroidie à +4°C afin d'accélérer d'avantage le refroidissement.
- Maintenir l'agitation jusqu'à ce que la température de l'émulsion soit inférieure à 25°C.

**C/Préparation du gel**

Le gel (phase C) peut être préparé à l'avance (pendant l'émulsification) :

Pour la composition 1 :

- Disperser le Carbomer dans l'eau pour le faire gonfler sous agitation modérée (Pâle défloculeuse, Rayneri - 700 rpm).
- Ajouter la triéthanolamine pour neutraliser le gel.

Pour la composition 2 :

- Ajouter l'eau de la phase C puis incorporer le copolymère d'acrylamide sous agitation modérée (Pâle défloculeuse, Rayneri - 700 rpm).

**D/Gélification des oléosomes**

- Après refroidissement de l'émulsion à température ambiante, ajouter si nécessaire de l'eau pour compenser les pertes.
- Agiter (Pâle défloculeuse, Rayneri - 200 rpm) jusqu'à homogénéisation.
- Ajouter progressivement le gel dans l'émulsion, sous agitation lente (Pâle défloculeuse, Rayneri
- 200 rpm), maintenir l'agitation jusqu'à obtention d'un mélange homogène (au besoin, monter l'agitation à 800 rpm maximum pendant 3 minutes pour aider à l'homogénéisation puis réduire à nouveau la vitesse).

**Exemple 3: Composition selon l'invention comprenant le composé A dans la phase aqueuse**

La composition 2 comprend les ingrédients suivants :

| Phase | Ingrédients | Teneur (%m/m) |
|---|---|---|
| A | Steareth-10 | 1.10 |
| A | Acide stéarique | 0.70 |
| A | Alcool stéarylique | 0.25 |
| A | Steareth-2 | 1.60 |
| A | Triglycérides d'acides caprique / caprylique | 6.00 |
| A | Cetostéaryl isononanoate | 6.00 |
| A | Cyclopentasiloxane | 3.00 |
| A | DL-alpha tocophérol | 0.20 |
| B | Composé A | 0.01 |
| B | Disodium édétate | 0.10 |
| B | Glycérine | 5.00 |
| B | Eau purifiée | 30.00 |
| B | Triéthanolamine | 0.20 |
| B | Phénoxyéthanol | 1.00 |
| B | Propylène glycol | 20.00 |
| B | Ethanol absolu | 10.00 |
| C | Carbomer 980 | 1.00 |
| C | Triéthanolamine | 1.20 |
| C | Eau purifiée | 12.65 |

Elle est préparée selon le protocole indiqué dans l'exemple 2 ii).

**Exemple 4 : Etude de stabilité de la composition 1' de l'exemple 2 et de la composition B de l'exemple 3**

Les compositions 1' de l'exemple 2 et 2 de l'exemple 3 ont été mises en stabilité à différentes conditions : 4°C,

température ambiante et 40°C pendant 3 mois.

La taille des olésosomes a été suivie au cours de l'étude de stabilité par DLS (Dynamic Light Scattering) au moyen d'un granulomètre de type Zetasizer Nano ZS (Malvern Instruments), après dilution des échantillons dans de l'eau distillée.

Le pH a été mesuré directement dans la composition.

Le composé A a été dosé par HPLC. Les résultats sont exprimés en mg/g.

Les résultats figurent dans le tableau ci-dessous.

| | **Composition 1' (phase huileuse)** | | | **Composition 2 (phase aqueuse)** | | |
|---|---|---|---|---|---|---|
| **Aspect microscopique** | Crème blanche, épaisse, lisse | | | Crème blanche, épaisse, lisse | | |
| **Dosage (mg/g)** | **TA** | **4°C** | **40°C** | **TA** | **4°C** | **40°C** |
| T0 | 0.48 | -- | -- | 104.0 | -- | -- |
| T1M | 0.049 | 0.047 | 0.049 | 102.0 | 100.6 | 102.6 |
| T2M | 0.049 | 0.048 | 0.047 | 105.5 | 105.6 | 104.7 |
| T3M | 0.048 | 0.47 | 0.048 | 102.6 | 103.3 | 111.0 |
| **pH** | | | | | | |
| T0 | 7.53 | -- | -- | 6.96 | -- | -- |
| T1M | 7.45 | 7.62 | 7.37 | 6.89 | 6.89 | 6.79 |
| T2M | 7.38 | 7.84 | 7.29 | NR | NR | NR |
| T3M | 7.42 | 7.81 | 7.27 | NR | NR | NR |
| NR: Non réalisé | | | | | | |

Conclusion:

Les résultats de stabilité montrent que le composé A est stable chimiquement 3 mois, à toutes les conditions de température, aussi bien lorsqu'il est solubilisé en phase aqueuse, que lorsqu'il est solubilisé dans les globules liquides huileux que sont les oléosomes.

**Exemple 5** : **Compositions selon l'invention comprenant le composé A**

Les compositions qui suivent sont préparées selon le mode opératoire indiqué dans l'exemple 2.

Leurs aspects macroscopique et microscopique à T0 sont étudiés afin de vérifier l'absence de recristallisation de l'actif, et le pH est mesuré.

Le composé A a été dosé par HPLC. Les résultats sont exprimés en pourcentage par rapport à la valeur initiale obtenue à T0.

Les stabilités physique et chimique sont évaluées à 1, 2, 3 et 6 mois, à température ambiante (TA) et à 40°C.

Les résultats obtenus figurent à la suite de chaque composition.

NR = Non Réalisé

i) Composition 3 :

| **Phase** | **Ingrédients** | **Teneur (%m/m)** |
|---|---|---|
| A | Steareth-10 | 1.25 |
| A | Acide stéarique | 0.85 |
| A | Alcool stéarylique | 0.25 |
| A | Steareth-2 | 1.80 |

(suite)

| Phase | Ingrédients | Teneur (%m/m) |
|---|---|---|
| A | Triglycérides d'acides caprique / caprylique | 15.50 |
| A | Phénoxyéthanol | 1.00 |
| A | DL-alpha tocophérol | 0.20 |
| A | Composé A | 0.01 |
| B | Cyclopentasiloxane | 4.00 |
| B | Disodium édétate | 0.10 |
| B | Glycérine | 5.00 |
| B | Eau purifiée | 35.46 |
| B | Triéthanolamine | 0.20 |
| B | Alcool benzylique | 0.40 |
| B | Chlorure de benzalkonium | 0.08 |
| C | Acrylamide / sodium acryloyldiméthyl taurate copolymère & isohexadécane & polysorbate 80 | 4.00 |
| C | Eau purifiée | 30.00 |

| | | | | | |
|---|---|---|---|---|---|
| **CARACTERISATION À T0** | | **ASPECT MACROSCOPIQUE** | Crème épaisse blanche brillante | | |
| | | **ASPECT MICROSCOPIQUE** | Tapis d'émulsion fin et homogène / Absence de cristaux d'actif | | |
| | | **pH** | 7.71 | | |
| | | **CENTRIFUGATION (5000RPM/ 30min)** | Conforme | | |
| | | | | | |
| **Suivi des stabilités** | | **1MOIS** | **2MOIS** | **3MOIS** | **6MOIS** |
| **pH TA/40°C** | | 7.73 / 7.60 | 7.72/7.43 | 7.77 / 7.21 | 7.72 / 6.97 |
| **Dosage (% actif relatif/ T0)** | **TA / 40°C** | 101.3 / 100.9 | 99.2 / 99.0 | 100.6/99.2 | 100.4 / 97.0 |
| Actif = composé A | | | | | |

ii) Composition 4

| Phase | Ingrédients | Teneur (%m/m) |
|---|---|---|
| A | Steareth-10 | 1.25 |
| A | Acide stéarique | 0.85 |
| A | Alcool stéarylique | 0.25 |
| A | Steareth-2 | 1.80 |
| A | Triglycérides d'acide caprique/ caprylique | 15.50 |
| A | Phénoxyéthanol | 1.00 |

(suite)

| Phase | Ingrédients | Teneur (%m/m) |
|---|---|---|
| A | DL-alpha tocophérol | 0.20 |
| A | Composé A | 0.01 |
| A | Cyclopentasiloxane | 4.00 |
| B | Glycérine | 5.00 |
| B | Chlorure de benzalkonium | 0.08 |
| B | Eau purifiée | 21.80 |
| B | Triethanolamine | 0.20 |
| C | Acrylamide / sodium acryloyldiméthyl taurate copolymère & isohexadécane & polysorbate 80 | 4.00 |
| C | Eau purifiée | 43.66 |
| C | Benzyl Alcohol | 0.40 |

<table>
<tr><td colspan="2" rowspan="4">CARACTERISATION À T0</td><td colspan="2">ASPECT MACROSCOPIQUE</td><td colspan="2">Crème épaisse blanche lisse</td></tr>
<tr><td colspan="2">ASPECT MICROSCOPIQUE</td><td colspan="2">Tapis d’émulsion fin et homogène / Absence de cristaux d’actif</td></tr>
<tr><td colspan="2">pH</td><td colspan="2">7.71</td></tr>
<tr><td colspan="2">CENTRIFUGATION (5000RPM/ 30min)</td><td colspan="2">Conforme</td></tr>
<tr><td colspan="6"></td></tr>
<tr><td colspan="2">Suivi des stabilités</td><td>1MOIS</td><td>2MOIS</td><td>3MOIS</td><td>6MOIS</td></tr>
<tr><td colspan="2">pH TA/40°C</td><td>7.73/7.60</td><td>7.72/7.43</td><td>7.77/7.21</td><td>7.72/6.97</td></tr>
<tr><td>Dosage (% actif relatif/ T0)</td><td>TA / 40°C</td><td>101.3% / 100.9%</td><td>99.2% / 99.0%</td><td>100.6% / 99.2%</td><td>100.4% / 97.0%</td></tr>
</table>

iii) Composition 5 :

| Phase | Ingrédients | Teneur (%m/m) |
|---|---|---|
| A | Steareth-10 | 1.12 |
| A | Acide stéarique | 0.76 |
| A | Alcool stéarylique | 0.25 |
| A | Steareth-2 | 1.62 |
| A | Triglycérides d’acides caprique / caprylique | 10.00 |
| A | Huile minérale | 4.00 |
| A | Huile de noix de coprah | 6.00 |
| A | Huile d’amande douce | 8.00 |
| A | Phénoxyéthanol | 1.00 |
| A | DL-alpha tocophérol | 0.20 |

(suite)

| Phase | Ingrédients | Teneur (%m/m) |
|---|---|---|
| A | Composé A | 0.01 |
| B | Glycérine | 10.00 |
| B | Eau purifiée | 31.04 |
| B | Sodium hydroxyde solution 1% | 3.50 |
| B | Alcool benzylique | 0.40 |
| B | Chlorure de benzalkonium | 0.10 |
| C | Acrylamide / sodium acryloyldiméthyl taurate copolymère & isohexadécane & polysorbate 80 | 2.00 |
| C | Eau purifiée | 20.00 |

| | | | | | |
|---|---|---|---|---|---|
| **CARACTERISATION À T0** | | **ASPECT MACROSCOPIQUE** | Crème épaisse jaunâtre lisse | | |
| | | **ASPECT MICROSCOPIQUE** | Tapis d'émulsion fin et homogène / Absence de cristaux d'actif | | |
| | | **pH** | 7.69 | | |
| | | **CENTRIFUGATION (5000RPM/ 30min)** | Conforme | | |
| | | | | | |
| **Suivi des stabilités** | | **1MOIS** | **2MOIS** | **3MOIS** | **6MOIS** |
| **pH TA/40°C** | | 7.57/7.34 | 7.59/7.22 | 7.56/7.05 | 7.61 / 6.74 |
| **Dosage (% actif relatif/ T0)** | **TA/ 40°C** | 101.6%/ 100% | 101%/99.6% | 100.3% / 99.7% | 100.1% / 98.6% |
| Actif = composé A | | | | | |

iv) Composition 6 :

| Phase | Ingrédients | Teneur (%m/m) |
|---|---|---|
| A | Steareth-10 | 1.12 |
| A | Acide stéarique | 0.76 |
| A | Alcool stéarylique | 0.25 |
| A | Steareth-2 | 1.62 |
| A | Triglycérides d'acides caprique / caprylique | 10.00 |
| A | Huile minérale | 4.00 |
| A | Huile de coprah | 6.00 |
| A | Huile d'amande douce | 8.00 |
| A | Phénoxyéthanol | 1.00 |
| A | DL-alpha tocophérol | 0.20 |
| A | Composé A | 0.03 |
| B | Glycérine | 10.00 |

(suite)

| Phase | Ingrédients | Teneur (%m/m) |
|---|---|---|
| B | Eau purifiée | 31.02 |
| B | Sodium hydroxyde solution 1% | 3.50 |
| B | Alcool benzylique | 0.40 |
| B | Chlorure de benzalkonium | 0.10 |
| C | Acrylamide / sodium acryloyldiméthyl taurate copolymère & isohexadécane & polysorbate 80 | 2.00 |
| C | Eau purifiée | 20.00 |

| | | |
|---|---|---|
| CARACTERISATION À T0 | ASPECT MACROSCOPIQUE | Crème épaisse jaunâtre lisse légère odeur de rance |
| | ASPECT MICROSCOPIQUE | Tapis d'émulsion fin et homogène / Absence de cristaux d'actif |
| | pH | 7.53 |
| | CENTRIFUGATION (5000RPM/ 30min) | Conforme |

v) Composition 7 :

| Phase | Ingrédients | Teneur (%m/m) |
|---|---|---|
| A | Steareth-10 | 1.25 |
| A | Acide stéarique | 0.85 |
| A | Alcool stéarylique | 0.25 |
| A | Steareth-2 | 1.80 |
| A | Triglycérides d'acides caprique / caprylique | 15.00 |
| A | Cyclopentasiloxane | 4.00 |
| A | Phénoxyéthanol | 1.00 |
| A | DL-alpha tocophérol | 0.20 |
| A | Composé A | 0.01 |
| B | Glycérine | 30.00 |
| B | Chlorure de benzalkonium | 0.08 |
| B | Eau purifiée | 8.06 |
| B | Sodium hydroxyde solution 1% | 3.50 |
| C | Acrylamide / sodium acryloyldiméthyl taurate copolymère & isohexadécane & polysorbate 80 | 4.00 |
| C | Eau purifiée | 30.00 |

| | | |
|---|---|---|
| CARACTERISATION À T0 | ASPECT MACROSCOPIQUE | Crème épaisse blanche brillante |
| | ASPECT MICROSCOPIQUE | Tapis d'émulsion fin et homogène / Absence de cristaux d'actif |
| | pH | 7.28 |
| | CENTRIFUGATION (5000RPM/ 30min) | Conforme |

vi) Composition 8 :

| Phase | Ingrédients | Teneur (%m/m) |
|---|---|---|
| A | Steareth-10 | 1.12 |
| A | Acide stéarique | 0.76 |
| A | Alcool stéarylique | 0.25 |
| A | Steareth-2 | 1.76 |
| A | Triglycérides d'acides caprique / caprylique | 15.00 |
| A | PPG-15 stéaryl éther | 8.50 |
| A | Huile minérale | 8.50 |
| A | Phénoxyéthanol | 1.00 |
| A | DL-alpha tocophérol | 0.20 |
| A | Composé A | 0.01 |
| B | Glycérine | 30.00 |
| B | Chlorure de benzalkonium | 0.08 |
| B | Eau purifiée | 16.82 |
| B | Sodium hydroxyde solution 1% | 4.00 |
| C | Acrylamide / sodium acryloyldiméthyl taurate copolymère & isohexadécane & polysorbate 80 | 2.00 |
| C | Eau purifiée | 10.00 |

| | | |
|---|---|---|
| CARACTERISATION ÀT0 | ASPECT MACROSCOPIQUE | Crème épaisse blanche brillante |
| | ASPECT MICROSCOPIQUE | Tapis d'émulsion fin et homogène / Absence de cristaux d'actif |
| | pH | 7.84 |
| | CENTRIFUGATION (5000RPM/ 30min) | Conforme |

vii) Composition 9 :

| Phase | Ingrédients | Teneur (%m/m) |
|---|---|---|
| A | Steareth-10 | 1.12 |

(suite)

| Phase | Ingrédients | Teneur (%m/m) |
|---|---|---|
| A | Acide stéarique | 0.76 |
| A | Alcool stéarylique | 0.25 |
| A | Steareth-2 | 1.62 |
| A | Huile de Ricin | 10.00 |
| A | PPG-15 stéaryl éther | 10.00 |
| A | Huile minérale | 8.50 |
| A | Phénoxyéthanol | 1.00 |
| A | DL-alpha tocophérol | 0.20 |
| A | Composé A | 0.01 |
| B | Glycérine | 30.00 |
| B | Chlorure de benzalkonium | 0.10 |
| B | Eau purifiée | 20.44 |
| B | Sodium hydroxyde solution 1% | 4.00 |
| C | Acrylamide / sodium acryloyldiméthyl taurate copolymère & isohexadécane & polysorbate 80 | 2.00 |
| C | Eau purifiée | 10.00 |

| | | |
|---|---|---|
| **CARACTERISATION À T0** | **ASPECT MACROSCOPIQUE** | Crème épaisse blanche brillante |
| | **ASPECT MICROSCOPIQUE** | Tapis d'émulsion fin et homogène / Absence de cristaux d'actif |
| | **CENTRIFUGATION (5000RPM/ 30min)** | Conforme |

viii) Composition 10:

| Phase | Ingrédients | Teneur (%m/m) |
|---|---|---|
| A | Steareth-10 | 1.25 |
| A | Acide stéarique | 0.85 |
| A | Alcool stéarylique | 0.25 |
| A | Steareth-2 | 1.80 |
| A | Triglycérides d'acide caprique/ caprylique | 15.00 |
| A | Phénoxyéthanol | 1.00 |
| A | DL-alpha tocophérol | 0.20 |
| A | Composé A | 0.01 |
| A | Cyclopentasiloxane | 4.00 |
| B | Glycérine | 20.00 |
| B | Chlorure de benzalkonium | 0.08 |

(suite)

| Phase | Ingrédients | Teneur (%m/m) |
|---|---|---|
| B | Eau purifiée | 23.06 |
| B | Sodium hydroxyde solution 1% | 3.50 |
| C | Acrylamide / sodium acryloyldiméthyl taurate copolymère & isohexadécane & polysorbate 80 | 4.00 |
| C | Eau purifiée | 25.00 |

| | | |
|---|---|---|
| **CARACTERISATION ÀT0** | **ASPECT MACROSCOPIQUE** | Crème épaisse blanche brillante |
| | **ASPECT MICROSCOPIQUE** | Tapis d'émulsion fin et homogène / Absence de cristaux d'actif |
| | **pH** | 7.34 |
| | **CENTRIFUGATION (5000RPM/ 30min)** | Conforme |

ix)

x) Composition 11

| xi) Phase | Ingrédients | Teneur (%m/m) |
|---|---|---|
| A | Steareth-10 | 1.25 |
| A | Acide stéarique | 0.85 |
| A | Alcool stéarylique | 0.25 |
| A | Steareth-2 | 1.80 |
| A | Triglycérides d'acide caprique/ caprylique | 15.00 |
| A | Phénoxyéthanol | 1.00 |
| A | DL-alpha tocophérol | 0.20 |
| A | Composé A | 0.03 |
| A | Cyclopentasiloxane | 4.00 |
| B | Glycérine | 20.00 |
| B | Chlorure de benzalkonium | 0.08 |
| B | Eau purifiée | 23.04 |
| B | Sodium hydroxyde solution 1% | 3.50 |
| C | Acrylamide / sodium acryloyldiméthyl taurate copolymère & isohexadécane & polysorbate 80 | 4.00 |
| C | Eau purifiée | 25.00 |

| | | |
|---|---|---|
| **CARACTERISATION À T0** | **ASPECT MACROSCOPIQUE** | Crème épaisse blanche brillante |
| | **ASPECT MICROSCOPIQUE** | Tapis d'émulsion fin et homogène / Absence de cristaux d'actif |
| | **pH** | 7.25 |
| | **CENTRIFUGATION (5000RPM/ 30min)** | Conforme |

**Exemple 6** : **Etude de tolérance après application unique sur l'Oreille de souris BALB/C.**

Le but de l'étude est d'évaluer l'effet d'une composition selon l'invention, sur l'activité pro-inflammatoire liée aux rétinoïdes de façon générale.

Une application unique de 20µl des compositions 1 et 2 a été administrée sur l'oreille des souris pendant 7 jours. Des observations cliniques et mesures de l'épaisseur de l'oreille de souris, directement reliée à l'inflammation, sont mesurées à partir du jour 2 et ce jusqu'au jour 19.

Différentes formulations ont été testées :

- la formulation oléosome contenant le composé A dans les globules liquides huileux correspondant à la composition 1 de l'exemple 2,
- la formulation oléosome contenant le composé A en phase aqueuse correspondant à la composition 2 de l'exemple 3,
- la formulation oléosome placebo correspondant à la formule placebo (sans composé A) de composition 2 de l'exemple 3.

Chaque formulation a été comparée à un groupe de souris non traité.

Résultats :

Le graphique de la Figure 1 décrit la mesure de l'épaisseur de l'oreille de la souris sur une durée de 19 jours.

A partir des valeurs de la Figure 1, l'aire sous la courbe traduisant l'augmentation de l'épaisseur de l'oreille de la souris a été calculée, pour obtenir la Figure 2.

Conclusion :

Même si une irritation spécifique des rétinoïdes est observée, la formulation oléosomes contenant le composé A dans les globules liquides huileux (composition 1) est moins irritante que la formulation oléosome comprenant le composé A dans la phase aqueuse (composition 2).

**Exemple 7 : Comparaison de la libération-pénétration cutanée in vitro du composé A formulé dans un gel de référence, avec la formulation oléosomes comprenant le composé A en phase aqueuse, et la formulation oléosomes comprenant le composé A dans les globules liquides huileux**

Conditions de l'étude :

Dans cette étude, les formulations ont été appliquées pendant 16h à la surface de la peau. A la fin de l'application, le composé A est quantifié dans les différents compartiments de la peau : stratum corneum, épiderme, derme et liquide récepteur selon une méthode de bioanalyse validée réalisée par spectrométrie de masse en tandem par ionisation electrospray positive, et utilisant un appareil Xevo (Waters). La limite de quantification pour le composé A est de 1ng/mL. Les conditions de LC/MS/MS mises au point ont permis de détecter jusqu'à 0,1% de la dose appliquée dans chacun des compartiments (dose non absorbée, stratum, épiderme, derme et liquide récepteur).

Les détails de l'application cutanée sont donnés dans le tableau ci-dessous.

| **Peau : 3 donneurs, 2 échantillons par donneur** | |
|---|---|
| Source | Peau humaine abdominale entière |
| Epaisseur | 0.79-1.22mm |
| Age | 39-64 ans. |
| Cellules de Franz | 2 cm² |
| Volume liquide récepteur | 3mL |
| Fonction barrière | Evaluée par détermination de la Perte Insensible en Eau, acceptable sauf contre-indication |
| **Formulations** | |
| Gel de référence contenant 100µg/g composé A | |
| Composition 1 contenant 100µg/g composé A correspondant à **l'Exemple 2** | |
| Composition 2 contenant 100µg/g composé A correspondant à **l'Exemple 3** | |
| **Application** | |
| Application | ~2mg/cm² |
| Quantité d'actif appliqué | 142~241ng/cm² |
| Temps d'exposition | 16h |
| **Dosage échantillons** | |
| Lavage compartiment donneur<br>Kleenex (permettant de retirer le surplus de produit)<br>1er strip | "Excès" / Dose non absorbée |
| Stratum corneum<br>(2-15 strips max)<br>Epiderme<br>Derme | Peau Totale |
| Liquide Récepteur | Dose absorbée |
| **Analyses** | **LC/UV et LC/MS** |
| Limite de quantification | 1ng/ml |

Peau Totale + Dose absorbée : Pénétration totale

Ensemble des échantillons : Balance des masses

La formule du gel de référence est la suivante :

| **Constituants** | **%** |
|---|---|
| Composé A | 0.01 |
| Propylene Glycol | 30.00 |
| Ethanol 95-96% | 67.99 |
| Klucel HF Pharma | 2.00 |

Résultats :

Les résultats sont présentés en Figure 3 (en ng/cm2) et en Figure 4 (% dose appliquée).

Conclusions :

- La pénétration totale de l'actif à partir des différentes formules est équivalente à celle du gel de référence:
  - Pour le gel de référence comprenant l'actif : 7,46±2.93%,
  - Pour la formulation oléosomes comprenant l'actif dans les globules liquides huileux : 6.50±1.14% de dose appliquée pénètre,
  - Pour la formulation oléosomes comprenant l'actif dans la phase aqueuse: 8,75±2.93% de dose appliquée

pénètre.

- La distribution tissulaire est similaire quelle que soit la composition:
  - Les plus fortes quantités d'actif dans le stratum corneum, de plus faibles quantités dans l'épiderme,
  - Quasiment aucune pénétration dans le derme.

Aussi, de façon surprenante, la formulation oléosomes comprenant le composé A dans les globules liquides huileux présente une bonne pénétration de l'actif, et ce malgré l'internalisation de l'actif et l'absence totale de propénétrant, et ce versus un gel de référence riche en propénétrant.

**Exemple 8** : **Cinétique de pénétration in vitro du composé A**

La cinétique de pénétration du composé A a été étudiée pendant 24h, avec des temps de collecte à 0,5 h, 1h, 3h, 6h et 24h. Des biopsies cutanées ont été placées dans des cellules de diffusion contenant du tampon phosphate et les produits ont été appliqués à une dose de 2mg/cm$^2$. Six réplicates (N = 2 pour 3 donneurs) ont été effectués pour chaque produit et chaque temps d'échantillonnage. Les 3 mêmes donneurs ont été utilisés pour tous les temps de collecte.
Les produits appliqués, contenant le composé A, sont le gel de référence tel que défini à l'exemple 7 ci-dessus et la composition selon l'exemple 2.
Les détails de l'application cutanée sont donnés dans le tableau ci-dessous :

| **Peau** | **3 donneurs, 2 échantillons par donneur par temps, n=6** |
|---|---|
| Source | Peau humaine abdominale dermatomée de cadavre |
| Epaisseur | 500µm |
| Age | Non Communiqué |
| Cellules de Franz | 1-2cm² |
| Volume Liquide Récepteur | Non Communiqué |
| Fonction Barrière | Evaluée par eau tritiée |
| **Formulations testées** | |
| Gel de référence contenant 100µg/g composé A<br>Composition 1 contenant 100µg/g composé A correspondant à **l'Exemple 2** | |
| Application Formule | ~2mg/cm² |
| Quantité actif appliquée | Entre 100-200ng/cm² |
| Temps d'exposition | Jusqu'à 24heures |
| **Dosage Echantillons** | |
| **Temps d'exposition** | **0.5, 1, 3, 6, 24h** |
| Lavage compartiment donneur<br>Kleenex (permettant de retirer le surplus de produit)<br>1er strip | "Excès" / Dose non absorbée |
| Stratum corneum (2-15 strips max)<br>Epiderme<br>Derme | Peau Totale<br>Pénétration totale<br>Balance des masses |
| Liquide Récepteur | Dose absorbée |
| **Analyses**<br>Limite de quantification | **LC/UV et LC/MS**<br>1ng/ml |

Deux critères principaux ont été utilisés pour classer les formulations. La pénétration dans la l'épiderme, exprimée ng/cm$^2$, a été calculée ainsi que l'aire sous la courbe de pénétration dans l'épiderme, calculée du temps T0 à 24 heures à partir des valeurs exprimées en ng/cm$^2$.
Les résultats sont présentés en Figure 5.

La formulation oléosomes comprenant le composé A dans les globules liquides huileux présente un profil typique de pénétration, avec une partie linéaire suivie par un plateau. Toutefois, le plateau de la pénétration cutanée du composé A a été atteint plus tard qu'avec le gel de référence, indiquant ainsi une libération contrôlée du composé A. En outre, la pénétration du composé A dans l'épiderme après l'application de la formulation oléosomes atteint la valeur de 8.37ng/$cm^2$, et est donc sensiblement plus élevée que celle du gel de référence. Par ailleurs, la valeur d'aire sous la courbe obtenue pour la pénétration du composé A dans l'épiderme est de 159.8ng/$cm^2$*h, sensiblement proche de celle du gel de référence (98.06ng/$cm^2$*h).

Conclusion: Ces études démontrent que la formulation oléosomes, comprenant le composé A dans les globules liquides huileux, présente un profil de pénétration proche de celui du gel de référence, permettant une exposition sensiblement proche de celle du gel de référence. Une quantité relativement plus élevée de composé A a, en revanche, été détectée dans l'épiderme après application de la formulation oléosomes, indiquant une localisation préférentielle du composé dans l'épiderme. Ainsi la formulation oléosomes comprenant le composé A dans les globules liquides huileux, a permis d'obtenir une libération contrôlée du composé A tout en maintenant la même quantité totale d'actif pénétrée.

## Revendications

**1.** Composition comprenant au moins l'acide 3"-tert-butyl-4'-(2-hydroxy-ethoxy)-4"-pyrrolidin-1-yl-[1,1';3',1"]-terphenyl-4-carboxylique en tant qu'actif pharmaceutique, **caractérisée en ce que** :

- ladite composition est une émulsion huile-dans-eau formée de globules liquides huileux pourvus chacun d'un enrobage cristal liquide lamellaire et dispersés dans une phase aqueuse,
- ledit rétinoïde est solubilisé soit dans les globules liquides huileux, soit dans la phase aqueuse de l'émulsion huile-dans-eau,
- chaque globule liquide huileux est unitairement enrobé d'une couche monolamellaire ou oligolamellaire obtenue à partir d'au moins un agent tensioactif lipophile, d'au moins un agent tensioactif hydrophile et d'au moins un tensioactif anionique distinct, et
- les globules liquides huileux enrobés ont un diamètre moyen inférieur à 800 nm.

**2.** Composition selon la revendication 1, **caractérisée en ce que** les globules huileux enrobés ont un diamètre moyen inférieur ou égal à 500 nm.

**3.** Composition selon l'une des revendications 1 à 2, **caractérisée en ce que** l'agent tensioactif lipophile présente une HLB comprise entre 2 et 5.

**4.** Composition selon la revendication 3, **caractérisée en ce que** l'agent tensioactif lipophile présentant une HLB comprise entre 2 et 5 est choisi parmi le distéarate de sucrose, le distéarate de diglycéryle, le tristéarate de tétraglycéryle, le décastéarate de décaglycéryle, le monostéarate de diglycérol, le monostéarate de sorbitane, le tristéarate de sorbitane, le monostéarate de 15 diéthylène glycol, l'ester de glycérol et d'acides palmitique et stéarique, le monostéarate polyoxyéthyléné comportant 2 motifs oxyéthylène, le stéaryl éther polyoxyéthyléné comportant 2 motifs oxyéthylène, le mono- et dibéhénate de glycéryle et le tétrastéarate de pentaérythritol.

**5.** Composition selon l'une des revendications 1 à 4, **caractérisée en ce que** l'agent tensioactif hydrophile présente une HLB comprise entre 8 et 12.

**6.** Composition selon la revendication 5, **caractérisée en ce que** l'agent tensioactif hydrophile présentant une HLB comprise entre 8 et 12 est choisi parmi le monostéarate de sorbitane polyoxyéthyléné 4 OE, le tristéarate de sorbitane polyoxyéthyléné 20 OE, le monostéarate polyoxyéthyléné 8 OE, le monostéarate d'hexaglycéryle, le monostéarate polyoxyéthyléné 10 OE, le stéaryl éther polyoxyéthyléné 10 OE, le distéarate polyoxyéthylèné 12 OE et le distéarate de méthylglucose polyoxyéthyléné 20 OE.

**7.** Composition selon l'une des revendications 1 à 6, **caractérisée en ce que** le tensioactif anionique distinct est un acide gras ou un ester d'acide gras, ledit acide gras comprenant au moins une chaîne grasse saturée ayant plus de 12 atomes de carbone, de préférence entre 16 et 22.

**8.** Composition selon l'une des revendications 1 à 7, **caractérisée en ce que** le tensioactif anionique distinct est choisi parmi l'acide stéarique, l'acide palmitique, l'acide arachidique et l'acide béhénique.

**9.** Composition selon l'une des revendications 1 à 8, **caractérisée en ce que** les tensioactifs sont mélangés dans les proportions suivantes pour enrober chaque globule liquide huileux :

- Tensioactif lipophile: 45-50% en poids du mélange ;
- Tensioactif hydrophile: 30-35% en poids du mélange ; et
- Tensioactif anionique distinct : 20-25% en poids du mélange.

**10.** Composition selon l'une des revendications 1 à 9, **caractérisée en ce que** l'acide 3"-tert-butyl-4'-(2-hydroxy-éthoxy)-4"-pyrrolidin-1-yl-[1, T';3',1"]-terphenyl-4-carboxylique est présent en quantité comprise entre 0,00001 et 0,3% en poids par rapport au poids total de la composition.

**11.** Composition selon l'une des revendications 1 à 10 **caractérisée en ce que** les globules liquides huileux comprennent au moins un solvant huileux choisi parmi les acides gras polyéthoxylés, les éthers de glycols, les triglycérides et huiles en contenant et les esters d'acides gras.

**12.** Composition selon l'une des revendications 1 à 11, **caractérisée en ce que** le rapport entre la quantité totale de tensioactifs et la quantité totale de phase huileuse constituant les globules liquides huileux est compris entre 10% et 25%.

**13.** Composition selon l'une des revendications 1 à 12, **caractérisée en ce que** l'acide 3"-tert-butyl-4'-(2-hydroxy-ethoxy)-4"-pyrrolidin-1-yl-[1,1';3',1"]-terphenyl-4-carboxylique est solubilisé dans les globules liquides huileux.

**14.** Composition selon la revendication 13, **caractérisée en ce qu'**elle contient moins de 0.5%, de préférence moins de 0.3%, de préférence moins de 0.1% en poids de propénétrant.

**15.** Composition selon la revendication 13, **caractérisée en ce qu'**elle est exempte de tout propénétrant.

**16.** Composition selon l'une des revendications 1 à 15, **caractérisée en ce qu'**elle se présente sous une forme adaptée pour une administration topique.

**17.** Composition selon l'une des revendications 1 à 16, pour son utilisation comme médicament.

**18.** Composition selon l'une des revendications 1 à 16 pour son utilisation dans le traitement des affections dermatologiques.

**19.** Composition selon la revendication 18 pour son utilisation dans le traitement des affections dermatologiques humaines choisies parmi :

1) les affections dermatologiques liées à un désordre de la kératinisation portant sur la différenciation et sur la prolifération cellulaire notamment pour traiter les acnés vulgaires, comédoniennes, polymorphes, rosacées, les acnés nodulokystiques, conglobata, les acnés séniles, les acnés secondaires telles que l'acné solaire, médicamenteuse ou professionnelle ;
2) les troubles de la kératinisation, notamment les ichtyoses, les états ichtyosiformes, l'ichtyose lamellaire, la maladie de Darrier, les kératodermies palmoplantaires, les leucoplasies, le pityriasis rubra pilaire et les états leucoplasiformes, le lichen cutané ou muqueux (buccal) ;
3) les affections dermatologiques avec une composante immuno-allergique inflammatoire, avec ou sans trouble de la prolifération cellulaire, et notamment toutes les formes de psoriasis, qu'il soit cutané, muqueux ou unguéal, et même le rhumatisme psoriasique, ou encore la dermatite atopique et les différentes formes d'eczema;
4) les désordres cutanés dus à une exposition aux rayonnements U.V. ainsi que pour réparer ou lutter contre le vieillissement de la peau, qu'il soit photo-induit ou chronologique ou pour réduire les pigmentations et les kératoses actiniques, ou toutes pathologies associées au vieillissement chronologique ou actinique, telle la xérose, les pigmentations et les rides ;
5) Toute condition liée à des proliférations dermiques ou épidermiques bénignes, qu'elles soient ou non d'origine virale telles que verrues vulgaires, les verrues planes , le molluscum contagiosum et l'épidermodysplasie verruciforme, les papillomatoses orales ou florides,;
6) les désordres dermatologiques tels que les dermatoses immunes comme le lupus érythémateux, les maladies immunes bulleuses et les maladies du collagène, telle la sclérodermie ;
7) les stigmates de l'atrophie épidermique et/ou dermique induite par les corticostéroïdes locaux ou systémiques,

ou toute autre forme d'atrophie cutanée,
8) les troubles de la cicatrisation, ou pour prévenir ou pour réparer les vergetures, ou encore pour favoriser la cicatrisation,
9) dans le traitement de toute affection d'origine fongique au niveau cutané tel que le tinea pedis et le tinea versicolor,
10) les désordres de la pigmentation, tel l'hyperpigmentation, le mélasma, l'hypopigmentation ou le vitiligo ;
11) les états cancéreux ou précancéreux, cutanés ou muqueux comme les kératoses actiniques, la maladie de Bowen, les carcinomes in-situ, le kératoacanthome et les cancers cutanés comme le carcinome basocellulaire (BCC), le carcinome spinocellulaire (SCC) et les lymphomes cutanés tek que le lymphome T.

**20.** Composition selon la revendication 19, pour son utilisation dans le traitement des affections dermatologiques choisies parmi l'acné, les ichtyoses, les états ichtyosiformes, l'hyperkératose palmoplantaire ou le psoriasis.

**21.** Utilisation de la composition selon l'une des revendications 1 à 18 pour améliorer la tolérance de l'acide 3''-tert-butyl-4'-(2-hydroxy-ethoxy)-4''-pyrrolidin-1-yl-[1,1';3',1'']-terphenyl-4-carboxylique.

**22.** Utilisation de la composition selon l'une des revendications 1 à 19 pour améliorer la pénétration cutanée de l'acide 3''-tert-butyl-4'-(2-hydroxy-ethoxy)-4''-pyrrolidin-1-yl-[1,1';3',1'']-terphenyl-4-carboxylique.

**23.** Utilisation de la composition selon l'une des revendications 1 à 19 pour obtenir une libération contrôlée de l'acide 3''-tert-butyl-4'-(2-hydroxy-ethoxy)-4''-pyrrolidin-1-yl-[1,1';3',1'']-terphenyl-4-carboxylique, **caractérisé en ce qu'**il est solubilisé dans les globules liquides huileux.

**24.** Utilisation d'une composition comprenant au moins un rétinoïde pour améliorer la tolérance du rétinoide, ladite composition étant une émulsion huile-dans-eau formée de globules liquides huileux pourvus chacun d'un enrobage cristal liquide lamellaire et dispersés dans une phase aqueuse, le rétinoïde étant solubilisé soit dans les globules liquides huileux, soit dans la phase aqueuse de l'émulsion huile-dans-eau, chaque globule liquide huileux étant unitairement enrobé d'une couche monolamellaire ou oligolamellaire obtenue à partir d'au moins un agent tensioactif lipophile, d'au moins un agent tensioactif hydrophile et d'au moins un tensioactif anionique distinct, et les globules liquides huileux enrobés ayant un diamètre moyen inférieur à 800 nm,

**25.** Utilisation d'une composition selon la revendication 23 pour améliorer la pénétration cutanée du rétinoïde, ladite composition ne contenant pas de propénétrant.

**26.** Utilisation d'une composition selon la revendication 23 pour obtenir une libération contrôlée du rétinoïde, **caractérisé en ce que** le rétinoïde est solubilisé dans les globules liquides huileux.

FIG. 1

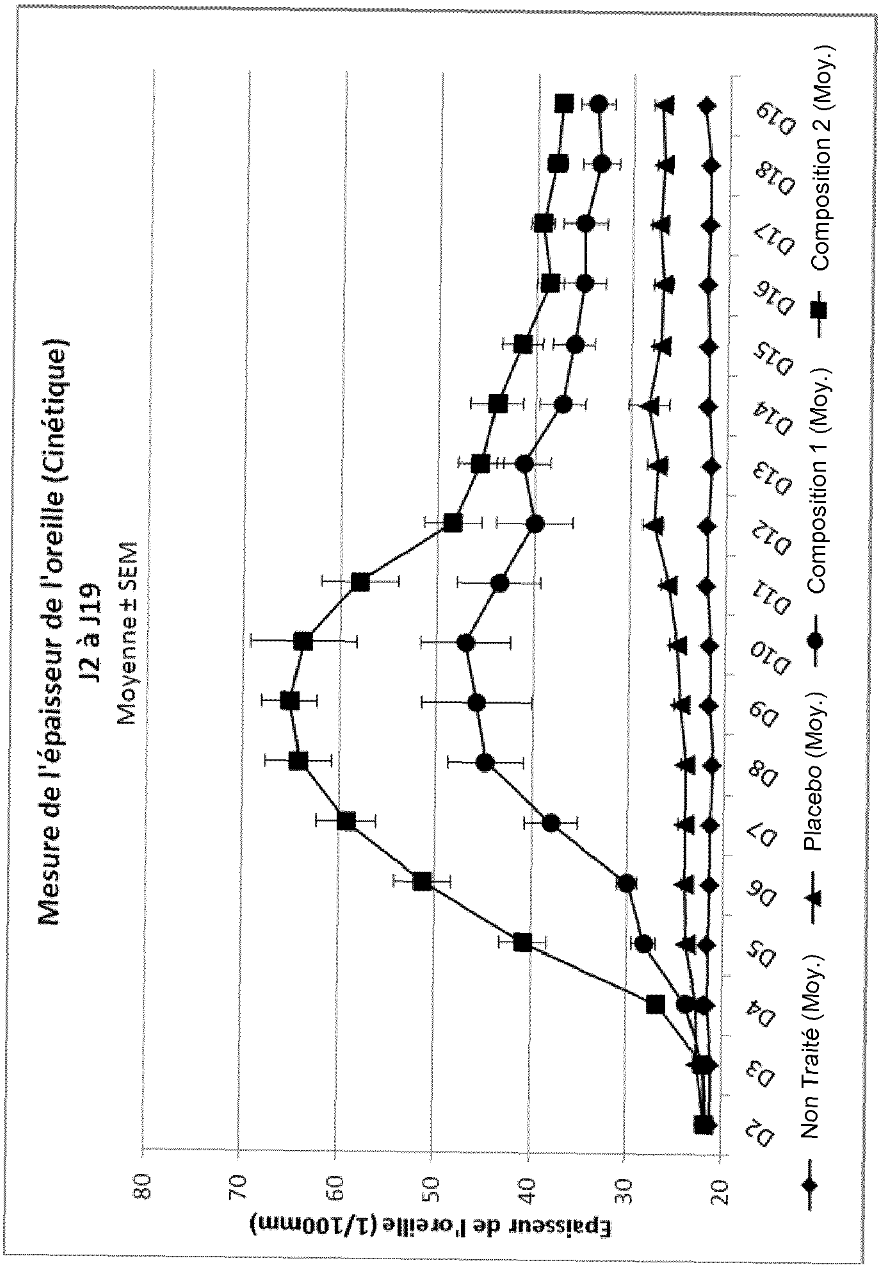
Mesure de l'épaisseur de l'oreille (Cinétique)
J2 à J19
Moyenne ± SEM
Epaisseur de l'oreille (1/100mm)
80
70
60
50
40
30
20
D2
D3
D4
D5
D6
D7
D8
D9
D10
D11
D12
D13
D14
D15
D16
D17
D18
D19
Non Traité (Moy.)
Placebo (Moy.)
Composition 1 (Moy.)
Composition 2 (Moy.)

FIG. 2

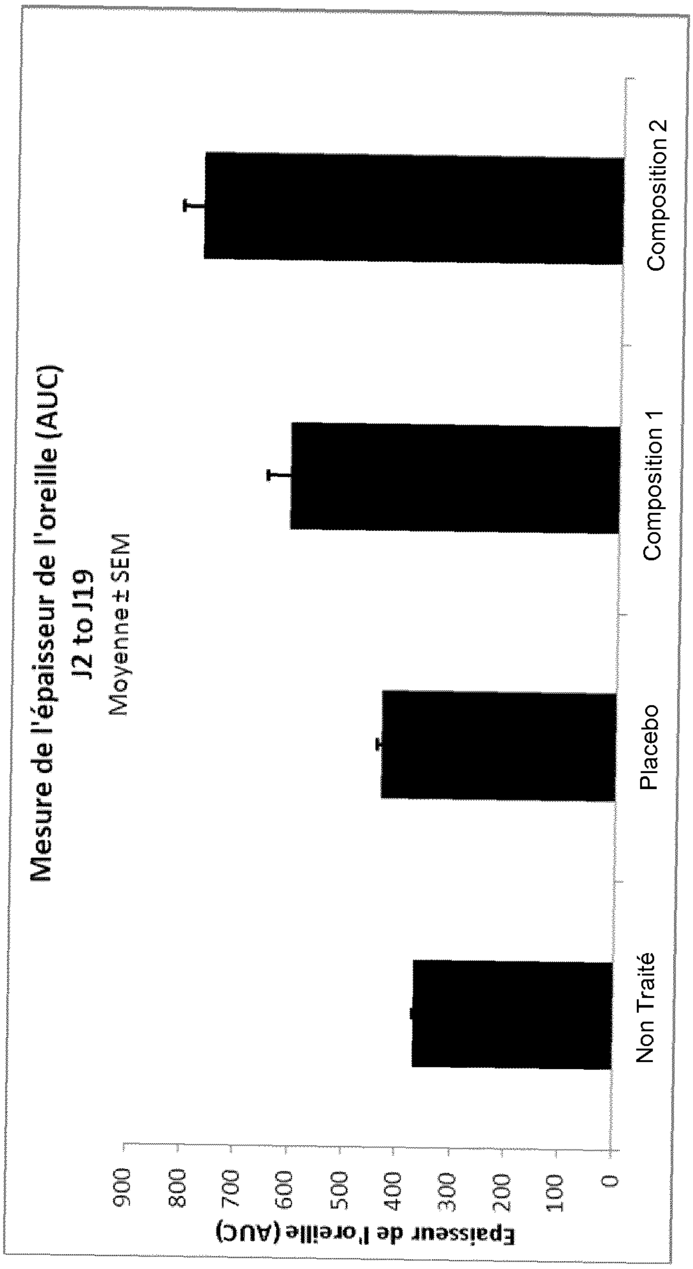
Mesure de l'épaisseur de l'oreille (AUC)
J2 to J19
Moyenne ± SEM
Epaisseur de l'oreille (AUC)
0
100
200
300
400
500
600
700
800
900
Non Traité
Placebo
Composition 1
Composition 2

FIG. 3

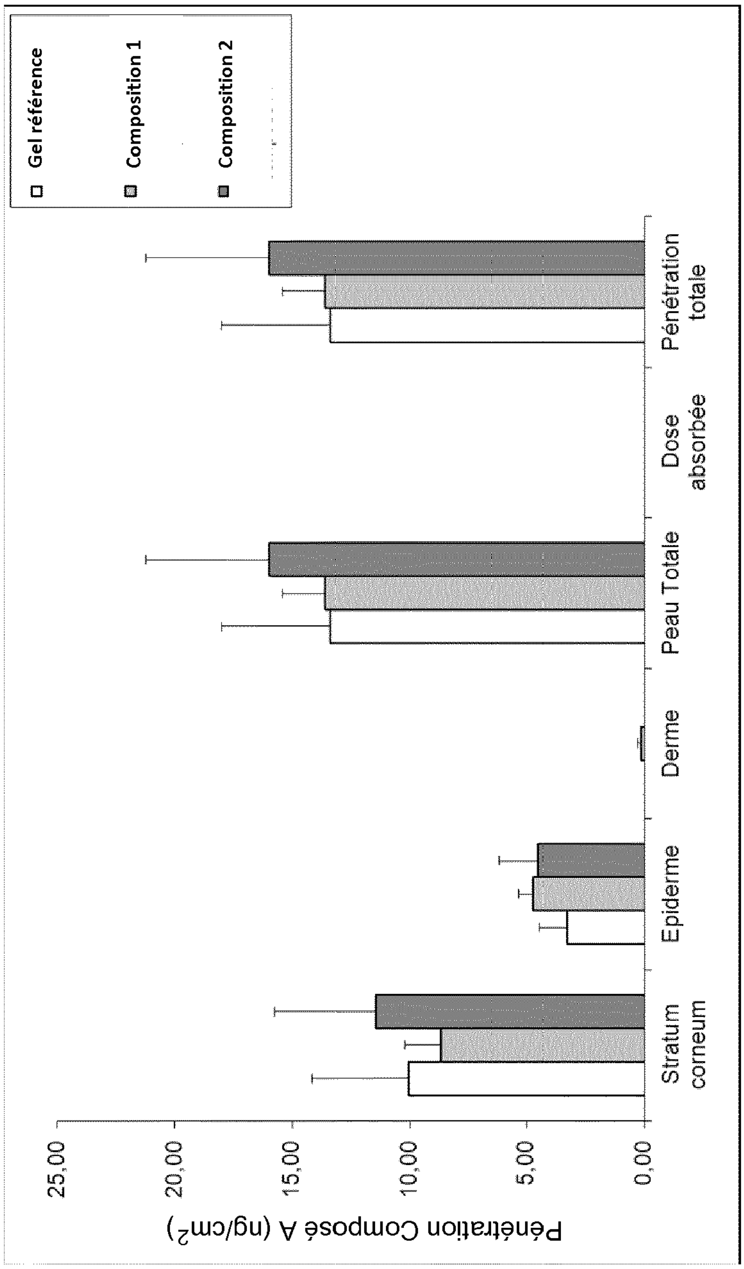
Gel référence
Composition 1
Composition 2
Pénétration Composé A (ng/cm²)
25,00
20,00
15,00
10,00
5,00
0,00
Stratum corneum
Epiderme
Derme
Peau Totale
Dose absorbée
Pénétration totale

FIG. 4

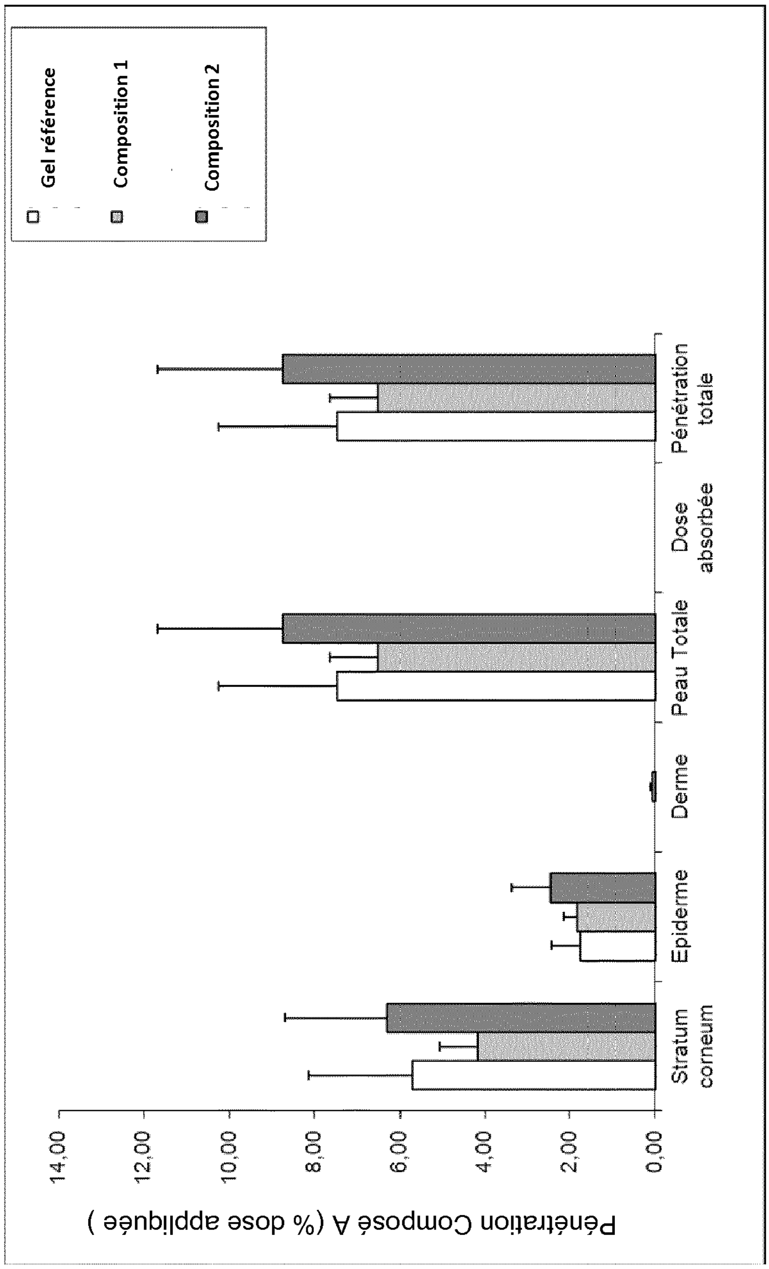
Pénétration Composé A (% dose appliquée )
14,00
12,00
10,00
8,00
6,00
4,00
2,00
0,00
Stratum corneum
Epiderme
Derme
Peau Totale
Dose absorbée
Pénétration totale
Gel référence
Composition 1
Composition 2

Europäisches Patentamt
European Patent Office
Office européen des brevets

# RAPPORT DE RECHERCHE EUROPEENNE

Numéro de la demande
EP 22 20 1948

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X,D<br>Y | EP 0 641 557 A1 (OREAL [FR])<br>8 mars 1995 (1995-03-08)<br>* colonne 5, lignes 29-32; revendications 1-14,21; exemples 2,6,13 *<br>----- | 24-26<br>1-20,22, 23 | INV.<br>A61K31/40<br>A61K31/402<br>A61K31/4015<br>A61K9/00<br>A61K9/107<br>A61P17/00 |
| Y,D | WO 2006/066978 A1 (GALDERMA RES & DEV [FR]; BIADATTI THIBAUD [FR]; DUMAIS LAURENCE [FR];) 29 juin 2006 (2006-06-29)<br>* exemple 25 *<br>----- | 1-23 | |
| Y | WO 2008/148968 A1 (GALDERMA RES & DEV [FR]; PELISSON ISABELLE [FR]; SUZIKI ITARU [FR]) 11 décembre 2008 (2008-12-11)<br>* revendication 2 *<br>----- | 1-23 | |
| X<br>Y | EP 0 900 559 A1 (OREAL [FR])<br>10 mars 1999 (1999-03-10)<br>* alinéas [0009], [0017] *<br>* revendications 1-18 *<br>----- | 24<br>21 | |
| A | WO 2010/072787 A2 (JOHNSON & JOHNSON CONSUMER FR [FR]; ODDOS THIERRY [FR] ET AL.) 1 juillet 2010 (2010-07-01)<br>* le document en entier *<br>----- | 1-26 | DOMAINES TECHNIQUES RECHERCHES (IPC)<br>A61K<br>A61P |

Le présent rapport a été établi pour toutes les revendications

1

| Lieu de la recherche | Date d'achèvement de la recherche | Examinateur |
|---|---|---|
| Munich | 17 mars 2023 | Young, Astrid |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.......................................................................................................
& : membre de la même famille, document correspondant

EPO FORM 1503 03.82 (P04C02)

Europäisches Patentamt
European Patent Office
Office européen des brevets

Numéro de la demande
EP 22 20 1948

## REVENDICATIONS DONNANT LIEU AU PAIEMENT DE TAXES

La présente demande de brevet européen comportait lors de son dépôt les revendications dont le paiement était dû.

☐ Une partie seulement des taxes de revendication ayant été acquittée dans les délais prescrits, le présent rapport de recherche européenne a été établi pour les revendications pour lesquelles aucun paiement n'était dû ainsi que pour celles dont les taxes de revendication ont été acquittées, à savoir les revendication(s):

☐ Aucune taxe de revendication n'ayant été acquittée dans les délais prescrits, le présent rapport de recherche européenne a été établi pour les revendications pour lesquelles aucun paiement n'était dû.

## ABSENCE D'UNITE D'INVENTION

La division de la recherche estime que la présente demande de brevet européen ne satisfait pas à l'exigence relative à l'unité d'invention et concerne plusieurs inventions ou pluralités d'inventions, à savoir:

**voir feuille supplémentaire B**

☐ Toutes les nouvelles taxes de recherche ayant été acquittées dans les délais impartis, le présent rapport de recherche européenne a été établi pour toutes les revendications.

☒ Comme toutes les recherches portant sur les revendications qui s'y prêtaient ont pu être effectuées sans effort particulier justifiant une taxe additionnelle, la division de la recherche n'a sollicité le paiement d'aucune taxe de cette nature.

☐ Une partie seulement des nouvelles taxes de recherche ayant été acquittée dans les délais impartis, le présent rapport de recherche européenne a été établi pour les parties qui se rapportent aux inventions pour lesquelles les taxes de recherche ont été acquittées, à savoir les revendications:

☐ Aucune nouvelle taxe de recherche n'ayant été acquittée dans les délais impartis, le présent rapport de recherche européenne a été établi pour les parties de la demande de brevet européen qui se rapportent à l'invention mentionnée en premier lieu dans les revendications, à savoir les revendications:

☐ Le present rapport supplémentaire de recherche européenne a été établi pour les parties de la demande de brevet européen qui se rapportent a l'invention mentionée en premier lieu dans le revendications (Règle 164 (1) CBE)

Europäisches Patentamt
European Patent Office
Office européen des brevets

# ABSENCE D'UNITÉ D'INVENTION
# FEUILLE SUPPLÉMENTAIRE B

Numéro de la demande
EP 22 20 1948

La division de la recherche estime que la présente demande de brevet européen ne satisfait pas à l'exigence relative à l'unité d'invention et concerne plusieurs inventions ou pluralités d'inventions, à savoir :

**1. revendications: 1-26**

**composition à base d'oléosomes comprenant le composé A, et différentes utilisations de ladite composition différentes utilisations d'une composition à base d'oléosomes pour améliorer la formulation et faciliter l'administration de rétinoïdes**

**1.1. revendications: 1-23**

**composition à base d'oléosomes comprenant le composé A, et différentes utilisations de ladite composition**

**1.2. revendications: 24-26**

**différentes utilisations d'une composition à base d'oléosomes pour améliorer la formulation et faciliter l'administration de rétinoïdes**

---

**Prière de noter que toutes les inventions mentionnées sous point 1, qui ne sont pas nécessairement liées par un concept inventif commun, ont pu être recherchées sans effort particulier justifiant une taxe additionnelle.**

EPO FORM P0402

## ANNEXE AU RAPPORT DE RECHERCHE EUROPEENNE RELATIF A LA DEMANDE DE BREVET EUROPEEN NO.

EP 22 20 1948

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

17-03-2023

| Document brevet cité au rapport de recherche | Date de publication | Membre(s) de la famille de brevet(s) | Date de publication |
|---|---|---|---|
| EP 0641557 A1 | 08-03-1995 | AT 141494 T | 15-09-1996 |
| | | BR 9403022 A | 02-05-1995 |
| | | CA 2131477 A1 | 08-03-1995 |
| | | CN 1108089 A | 13-09-1995 |
| | | DE 69400400 T2 | 19-12-1996 |
| | | EP 0641557 A1 | 08-03-1995 |
| | | ES 2094029 T3 | 01-01-1997 |
| | | FR 2709666 A1 | 17-03-1995 |
| | | HU 215115 B | 28-09-1998 |
| | | JP 2665459 B2 | 22-10-1997 |
| | | JP H07165530 A | 27-06-1995 |
| | | PL 304928 A1 | 20-03-1995 |
| | | RU 2124884 C1 | 20-01-1999 |
| | | US 5658575 A | 19-08-1997 |
| WO 2006066978 A1 | 29-06-2006 | AU 2005318292 A1 | 29-06-2006 |
| | | BR PI0517500 A | 07-10-2008 |
| | | CA 2590246 A1 | 29-06-2006 |
| | | CY 1112721 T1 | 10-02-2016 |
| | | EP 1831149 A1 | 12-09-2007 |
| | | JP 5258299 B2 | 07-08-2013 |
| | | JP 2008525364 A | 17-07-2008 |
| | | KR 20070087626 A | 28-08-2007 |
| | | NL 301042 I1 | 17-06-2020 |
| | | PL 1831149 T3 | 29-06-2012 |
| | | WO 2006066978 A1 | 29-06-2006 |
| WO 2008148968 A1 | 11-12-2008 | AU 2008259754 A1 | 11-12-2008 |
| | | BR PI0809880 A2 | 30-09-2014 |
| | | CA 2685482 A1 | 11-12-2008 |
| | | CN 101674818 A | 17-03-2010 |
| | | EP 2167061 A1 | 31-03-2010 |
| | | FR 2915682 A1 | 07-11-2008 |
| | | JP 2010526124 A | 29-07-2010 |
| | | KR 20100016204 A | 12-02-2010 |
| | | RU 2009144988 A | 10-06-2011 |
| | | US 2010143445 A1 | 10-06-2010 |
| | | US 2012282314 A1 | 08-11-2012 |
| | | WO 2008148968 A1 | 11-12-2008 |
| EP 0900559 A1 | 10-03-1999 | BR 9802796 A | 11-01-2000 |
| | | DE 69800464 T2 | 10-05-2001 |
| | | EP 0900559 A1 | 10-03-1999 |
| | | ES 2155279 T3 | 01-05-2001 |
| | | FR 2767691 A1 | 05-03-1999 |
| | | JP H11139956 A | 25-05-1999 |

EPO FORM P0460

Pour tout renseignement concernant cette annexe : voir Journal Officiel de l'Office européen des brevets, No.12/82

## ANNEXE AU RAPPORT DE RECHERCHE EUROPEENNE RELATIF A LA DEMANDE DE BREVET EUROPEEN NO.

EP 22 20 1948

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

17-03-2023

| Document brevet cité au rapport de recherche | Date de publication | Membre(s) de la famille de brevet(s) | Date de publication |
|---|---|---|---|
| | | JP 2002179555 A | 26-06-2002 |
| | | KR 19990023818 A | 25-03-1999 |
| | | US 2001031273 A1 | 18-10-2001 |
| WO 2010072787 A2 | 01-07-2010 | AU 2009331482 A1 | 07-07-2011 |
| | | BR PI0923534 A2 | 26-04-2016 |
| | | CA 2747939 A1 | 01-07-2010 |
| | | CN 102256666 A | 23-11-2011 |
| | | EP 2376198 A2 | 19-10-2011 |
| | | ES 2665018 T3 | 24-04-2018 |
| | | KR 20110118778 A | 01-11-2011 |
| | | RU 2011130536 A | 27-01-2013 |
| | | US 2011251273 A1 | 13-10-2011 |
| | | WO 2010072787 A2 | 01-07-2010 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- FR 2894820
- US 5955109 A
- US 5650171 A
- WO 2006066978 A
- FR 0512367
- WO 0556516 A
- EP 04014809 W
- FR 2861069
- EP 0641557 A